# EUROPEAN PATENT APPLICATION

(11) **EP 4 667 551 A1**
(43) Date of publication of application: **24.12.2025**
(21) Application number: 24382656.7
(22) Date of filing: 18.06.2024
(51) Int. Cl.: C10G 1/10, C07C 11/06, C10G 3/00, C10G 11/05, C10G 51/02

(54) **A METHOD FOR PRODUCING PROPYLENE**

(71) Applicant: Neste Oyj, 02150 Espoo (FI)
(72) Inventor: CORMA CANOS, Avelino, 46022 Avda. Los Naranjos, s/n (Edificio 6C) Valencia (ES); TORRES MARTÍ, Ferran, 46022 Avda. Los Naranjos, s/n (Edificio 6C) Valencia (ES); MATHIEU, Yannick, 46022 Avda. Los Naranjos, s/n (Edificio 6C) Valencia (ES); MÄKELÄ, Eveliina, Box 310 06101, Porvoo (FI); ALBERSBERGER, Sylvia, Box 310 06101, Porvoo (FI); IMBAO, Jerick, Box 310 06101, Porvoo (FI); KURKIJÄRVI, Antti, P.O. Box 310 06101, Porvoo (FI); TIITTA, Marja, Box 310 06101, Porvoo (FI)
(74) Representative: Laine IP Oy

(57) **Abstract**

The present disclosure relates to a method for producing propylene D, in particular to methods comprising subjecting a sustainable hydrocarbon feed A to a first catalytic cracking 20 in the presence of a first cracking catalyst comprising a first zeolite, followed by a second catalytic cracking 30 in the presence of a second cracking catalyst comprising a second zeolite. The first and the second zeolite are selected from zeolites of a 10 MR framework type, hierarchical zeolites of a 10 MR framework type and zeolites of a 12 MR framework type, and the second zeolite has a framework type that is different from the first zeolite unless both the first and the second zeolite are hierarchical zeolites of a 10 MR framework type, with the proviso that when the first zeolite is a zeolite of a 10 MR framework type, the second zeolite is a hierarchical zeolite of a 10 MR framework type or a zeolite of a 12 MR framework type.

## Description

### TECHNICAL FIELD

The present disclosure relates to methods for producing propylene, in particular to methods comprising two-stage catalytic cracking of a feed containing sustainable hydrocarbons.

### BACKGROUND

Petrochemicals are a growing business area and propylene is the second most important starting material in the petrochemical industry after ethylene. Nearly two thirds of all demand of propylene is used for manufacturing of polypropylene.

There is an increasing demand to use sustainable polyethene and polypropene. However, there is very limited production capacity available for producing the sustainable chemicals that are used in polymerization processes. Renewable ethylene is available for example via carbohydrate fermentation to ethanol followed by catalytic dehydrogenation, but for propylene the production routes are more limited. Steam cracking of naphtha cannot meet the demand alone, even if sustainable naphtha was available in large volumes, because steam cracking provides propylene only as a side-product. Fluid catalytic cracking (FCC) is expected to be one of the main sources of propylene. Propylene is also manufactured from propane via dehydrogenation.

WO2009130392 discloses one-stage catalytic cracking of hydrogenated natural fats to C2-C8 hydrocarbons at 250-450 °C, using a catalyst based on a zeolite and a mesoporous inorganic oxide.

WO22096781A1 discloses one-stage catalytic cracking of highly paraffinic feedstocks, such as renewable feedstocks, using relatively low temperature and conventional cracking catalyst such as ZSM-5 to produce propylene and C4 olefin compositions, with an optional recycle of unconverted feedstock.

WO22096782A1 discloses one-stage catalytic cracking, particularly FCC, of highly paraffinic feedstocks, such as renewable feedstocks, using higher temperature and conventional FCC cracking catalyst to produce propylene and gasoline range compositions, with an optional recycle of unconverted feedstock.

WO2021204818A1 discloses a process to produce ethylene and propylene, where a hydrocarbon stream comprising pyrolysis plastic oil is upgraded by subjecting to a selective hydrogenation, followed by subjecting, preferably as diluted, to cracking using a cracking catalyst, which is a 10 MR and/or 12 MR molecular sieve, at a temperature ranging from 450 °C to 650 °C, a total pressure ranging from 0.5 to 10 barg and/or with a hydrogen partial pressure ranging from 0 to 7.5 barg, to crack the olefins and/or paraffins of the pyrolysis plastic oil into olefins having 2 to 4 carbon atoms, with an optional recycle of hydrocarbons having 4 to 6 carbon atoms. Part of the upgraded stream containing paraffins is further used in steam cracking process.

US5292976 discloses a staged process for conversion of naphtha. In a first stage, n-paraffins in naphtha are converted to aromatics over modified non-acidic zeolite catalyst particles with a low conversion of naphthenes in the feedstream. The effluent from the first stage is cascaded to a second stage reactor containing acidic zeolite catalyst wherein naphthenes are converted to light olefins. The process is said to result in a reduction in the production of light C1-C4 paraffins compared to earlier processes. The preferred catalyst for the first stage is a platinum modified zeolite containing tin.

WO20074693 discloses a staged process for producing propylene where a hydrocarbon feed optionally in admixture with a recycle stream is fed to a first fixed bed reactor, at 450-750 °C, and contacted with a low acidic density cracking catalyst at a hydrocarbon partial pressure of below 3 bar and at a WHSV of 0.5-100 h-1. Propylene and optionally other low boiling compounds are isolated from the first reactor effluent, whereafter all or part of the high boiling fraction optionally in admixture with a recycle stream is fed to a second fixed bed reactor, at 450-750 °C, and contacted with a high acidic density cracking catalyst at a hydrocarbon partial pressure of below 3 bar and at a WHSV of 0.5-100 h-1. The temperature of the feed fed to the first reactor is lower than the temperature of the feed fed to the second reactor. This is said to provide propylene in a high yield while the coke formation on the catalyst is kept at a rate such that an acceptable cycle length results. WO21206730 discloses a similar process but additionally recycling all or part of the high boiling fraction of the second reactor effluent to the first and/or to the second reactor.

Global warming and vanishing crude oil reserves generate a continued need for further industrial processes to manufacture sustainable chemicals usable in the petroleum sector, particularly approaches usable for converting varying feed compositions.

### SUMMARY

It is an aim to solve or alleviate at least some of the problems related to prior art, including reducing GHG emissions and dependence from petroleum sources, especially in the petrochemicals sector. More specifically, an aim is to provide a method for sustainable propylene production having improved flexibility regarding usable sustainable feeds. A further aim is to provide sustainable propylene usable in the petrochemicals sector in improved yields.

It is an object of the present disclosure to provide a method for producing propylene, the method comprising the following steps:
a) providing a sustainable hydrocarbon feed;
b) subjecting a first cracking feed comprising the sustainable hydrocarbon feed to catalytic cracking reaction in the presence of a first cracking catalyst comprising a first zeolite at a temperature within a range from 300 to 700 °C, preferably from 400 to 650 °C, to obtain a first catalytically cracked stream;
c) optionally separating from the first catalytically cracked stream at least a lighter fraction comprising at least C1-C3 hydrocarbons and a heavier fraction;
d) subjecting a second cracking feed comprising at least a portion, preferably the heavier fraction, of the first catalytically cracked stream to catalytic cracking reaction in the presence of a second cracking catalyst comprising a second zeolite at a temperature within a range from 350 to 750 °C, preferably from 450 to 700 °C, to obtain a second catalytically cracked stream; and
e) recovering from the second catalytically cracked stream, and optionally from the lighter fraction of the first catalytically cracked stream, at least a fraction rich in propylene;
wherein:
the first zeolite is selected from a group consisting of zeolites of a framework type having 10-membered ring as the largest ring-size (10 MR framework type), hierarchical zeolites of a 10 MR framework type, and zeolites of a framework type having 12-membered ring as the largest ring-size (12 MR framework type), preferably from hierarchical zeolites of a 10 MR framework type and zeolites of a 12 MR framework type,
the second zeolite is selected from a group consisting of zeolites of a 10 MR framework type, hierarchical zeolites of a 10 MR framework type, and zeolites of a 12 MR framework type, and
the second zeolite has a framework type that is different from the first zeolite unless both the first and the second zeolite are hierarchical zeolites of a 10 MR framework type,
with the proviso that when the first zeolite is a zeolite of a 10 MR framework type, the second zeolite is a hierarchical zeolite of a 10 MR framework type or a zeolite of a 12 MR framework type.

The present method provides various benefits as explained hereinafter and/or demonstrated in the experiments.

A number of exemplifying and non-limiting embodiments of the invention are described in accompanied dependent claims.

Various exemplifying and non-limiting embodiments of the invention together with additional objects and advantages thereof, will be best understood from the following description of specific exemplifying and non-limiting embodiments when read in connection with the accompanying figures.

The verbs "to comprise" and "to include" are used in this document as open limitations that neither exclude nor require the existence of also un-recited features. The features recited in dependent claims are mutually freely combinable unless otherwise explicitly stated. Furthermore, it is to be understood that the use of "a" or "an", i.e., a singular form, throughout this document does not exclude a plurality.

### BRIEF DESCRIPTION OF THE FIGURES

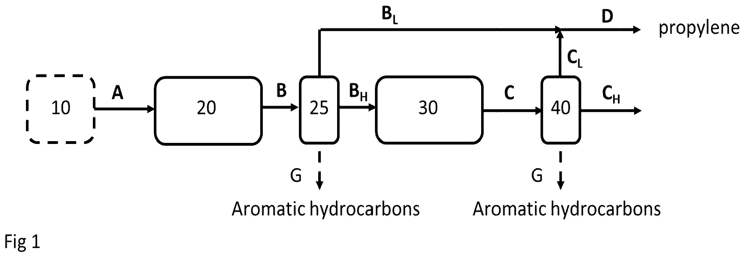

Figure 1 shows an exemplary non-limiting schematic overview of production of propylene according to an embodiment of the method of the present disclosure, including separating the first catalytically cracked stream at least to a heavier fraction fed to the second cracking stage and to a lighter fraction wherefrom, optionally as combined with a lighter fraction of the second catalytically cracked stream, at least propylene is recovered.

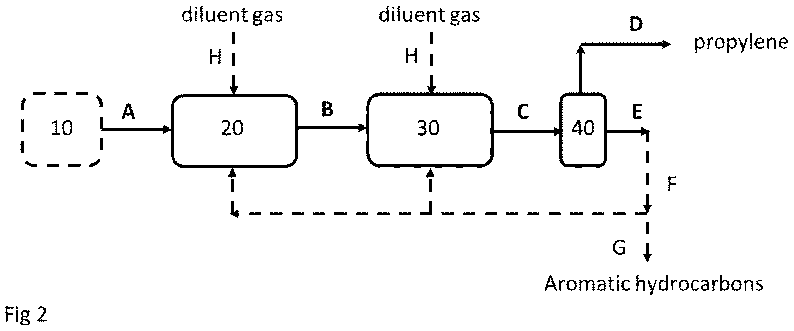

Figure 2 shows an exemplary non-limiting schematic overview of production of propylene according to an embodiment of the method of the present disclosure, with an optional diluent gas addition and optional recycling of C4+ hydrocarbons.

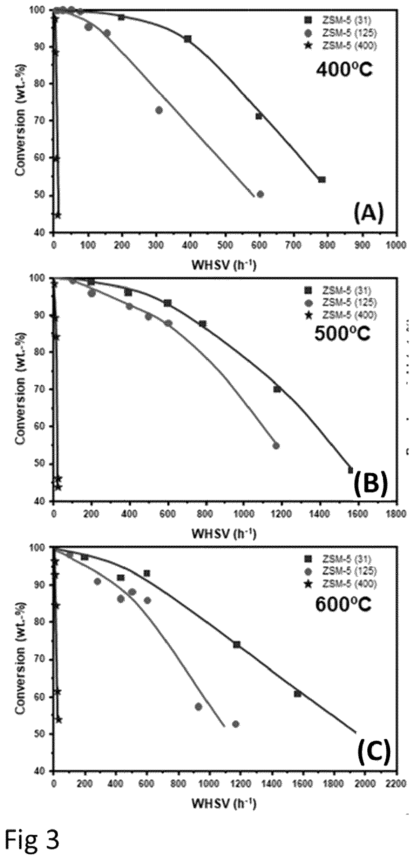

Figure 3 illustrates influence of Si/AI molar ratio of ZSM-5 over catalyst activity and propylene selectivity at A: 400 ºC, B: 500 °C, and C: 600 ºC when cracking n-hexadecane at total pressure of 1 atm, feed's partial pressure of 0.33 atm, and varying WHSV. The number in the brackets following the name of the zeolite indicates its Si/AI molar ratio.

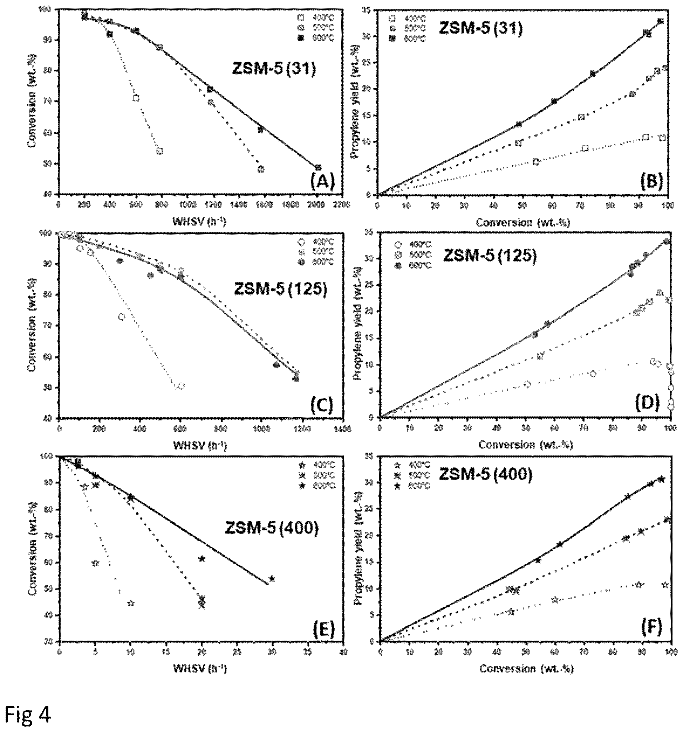

Figure 4 illustrates influence of cracking temperature over catalyst activity and propylene selectivity for ZSM-5 catalysts having different Si/AI molar ratios of 31 (A and B), 125 (C and D) and 400 (E and F), when cracking n-hexadecane at total pressure of 1 atm, feed's initial partial pressure of 0.33 atm, and varying WHSV.

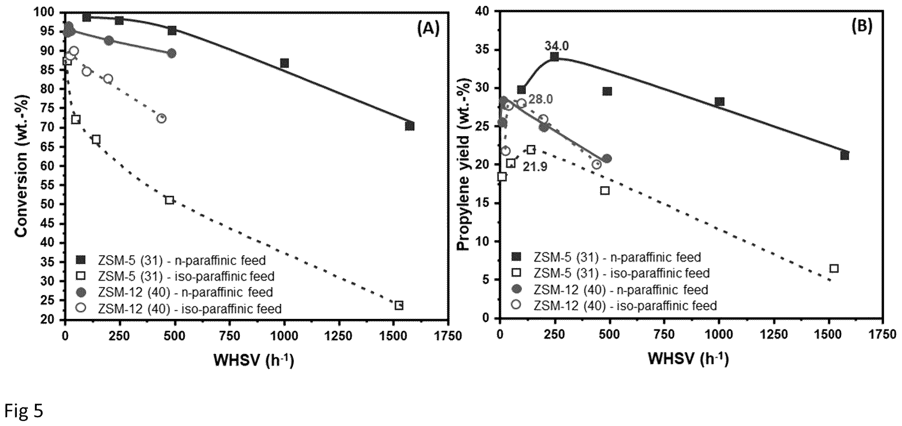

Figure 5 illustrates influence of WHSV over cracking activity in terms of conversion (A) and propylene yield (B) for ZSM-5 (31) and ZSM-12 (40) catalysts when cracking n-paraffinic or isomerized feed at 600 °C, atmospheric pressure, and feed's initial partial pressure of 0.33 atm. The number in the brackets following the name of the zeolite indicates its Si/AI molar ratio.

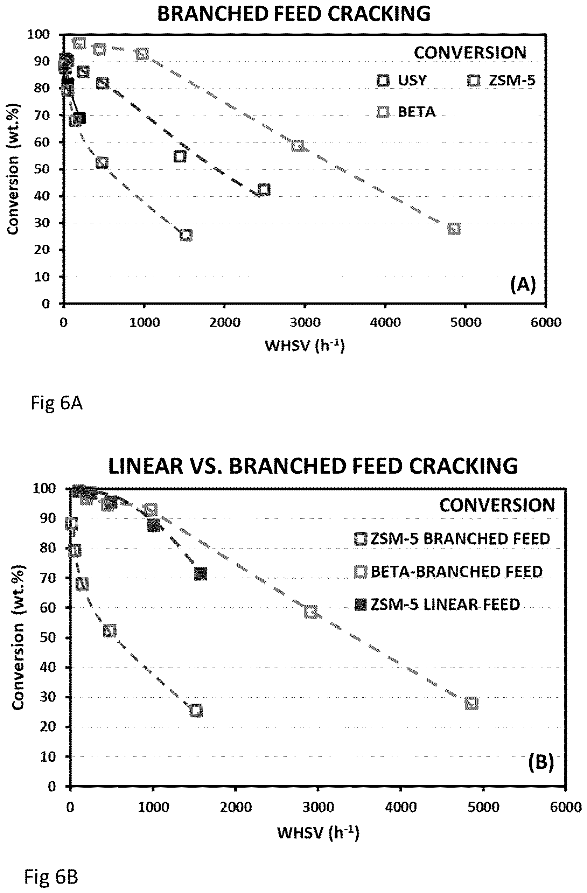

Figure 6A shows comparison of catalytic activity of zeolite beta, USY and ZSM-5 catalysts when cracking isomerized feed at 600 °C, atmospheric pressure, and feed's initial partial pressure of 0.33 atm, while Figure 6B shows catalytic activity of zeolite beta and ZSM-5 when cracking isomerized feed vs n-paraffinic feed, at the same conditions.

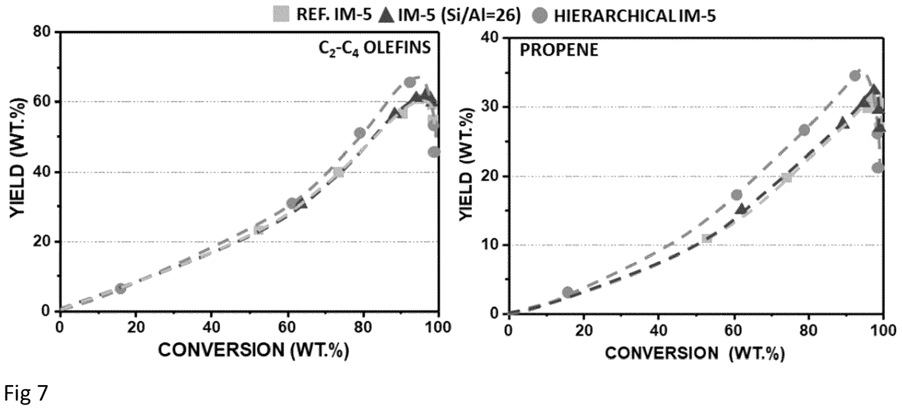

Figure 7 shows comparison of catalytic activity in terms of conversion and yields of propylene and light olefins (C2-C4) yield for hierarchical IM-5, the parent IM-5, and an otherwise similar IM-5 but having higher Si/SI molar ratio, when cracking n-paraffinic feed at 600 °C, atmospheric pressure, and feed's initial partial pressure of 0.33 atm.

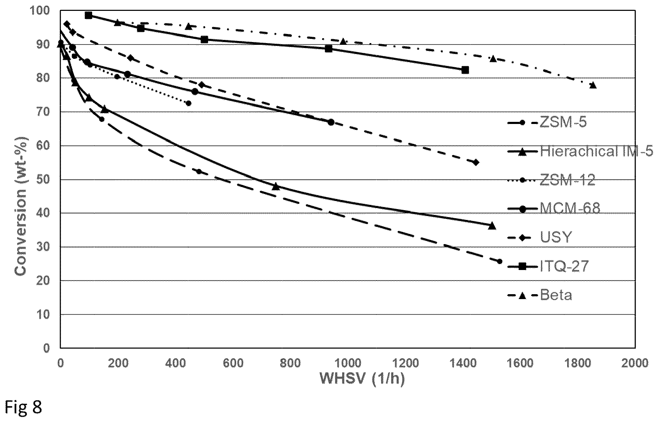

Figure 8 shows comparison of catalytic activity in terms of conversion for zeolites beta, ITQ-27, MCM-68, USY, ZSM-12, hierarchical IM-5 and ZSM-5, when cracking isoparaffinic feed at 600 °C, atmospheric pressure, and feed's initial partial pressure of 0.33 atm, and varying WHSV.

### DETAILED DESCRIPTION

In the following description, like reference signs denote like elements or steps. All standards referred to herein are the latest revisions available at the filing date, unless otherwise mentioned.

As used herein, hydrocarbons refer to compounds consisting of carbon and hydrogen, including paraffins, n-paraffins, i-paraffins, monobranched i-paraffins, multibranched i-paraffins, olefins, naphthenes, and aromatics. Oxygenated hydrocarbons refer herein to hydrocarbons comprising covalently bound oxygen.

As used herein, paraffins refer to non-cyclic alkanes, i.e. non-cyclic, open chain saturated hydrocarbons that are linear (normal paraffins, n-paraffins) or branched (isoparaffins, i-paraffins). In other words, paraffins refer herein to n-paraffins and/or i-paraffins.

As used herein, cyclic hydrocarbons refer to all hydrocarbons containing cyclic structure(s), including cyclic olefins, naphthenes, and aromatics. Naphthenes refer herein to cycloalkanes, i.e. saturated hydrocarbons containing at least one cyclic structure, with or without side chains. As naphthenes are saturated compounds, they are compounds without aromatic ring structure(s) present. Aromatics refer herein to hydrocarbons containing at least one aromatic ring structure, i.e. cyclic structure having delocalized, alternating π bonds all the way around said cyclic structure.

Unless otherwise stated, in the context of the present disclosure, for compositions boiling at 36 °C or higher at standard atmospheric pressure, contents of n-paraffins, i-paraffins, monobranched i-paraffins, multibranched isoparaffins, naphthenes, and aromatics are expressed as weight-% relative to the degassed weight of the composition in question, or when so defined, as weight-% relative to the total weight of paraffins, or total weight of i-paraffins of the composition in question. Said contents may be determined by GCxGC-FID/GCxGC-MS method, preferably conducted as follows: GCxGC (2D GC) method was run as generally disclosed in UOP 990-2011 and by Nousiainen M. in the experimental section of his Master's Thesis Comprehensive two-dimensional gas chromatography with mass spectrometric and flame ionization detectors in petroleum chemistry, University of Helsinki, August 2017, with the following modifications. The GCxGC was run in reverse mode, using a semipolar column (Rxi17Sil) first and a non-polar column (Rxi5Sil) thereafter, followed by FID detector, using run parameters: carrier gas helium 31.7 cm/s (column flow at 40 ºC 1.60 ml/min); split ratio 1:350; injector 280 ºC; column T program 40 ºC (0 min) - 5 ºC/min - 250 ºC (0 min) - 10 ºC/min - 300 ºC (5 min), run time 52 min; modulation period 10 s; detector 300 ºC with H₂ 40 ml/min and air 400 ml/min; makeup flow helium 30 ml/min; sampling rate 250 Hz and injection size 0.2 microliters. Individual compounds were identified using GCxGC-MS, with MS-parameters: ion source 230 ºC; interface 300 ºC; scan range 25 - 500 amu; event time (sec) 0.05; scan speed 20000. Commercial tools (Shimadzu's LabSolutions, Zoex's GC Image) were used for data processing including identification of the detected compounds or hydrocarbon groups, and for determining their mass concentrations by application of response factors relative to n-heptane to the volumes of detected peaks followed by normalization to 100 wt.-%. Olefins were lumped with naphthenes and heteroatomic species with aromatics, unless separately reported. The limit of quantitation for individual compounds of this method is 0.1 wt.-%.

In the context of this disclosure, CX+ hydrocarbons, paraffins, or similar, refer to hydrocarbons, paraffins, or similar, respectively, having a carbon number of at least X, where X is any feasible integer. It is understood that every compound falling within the definition is not necessarily present.

Unless otherwise stated, regarding distillation characteristics, such as initial boiling points (IBP), final boiling points (FBP), T5 temperature (5 vol-% recovered), T95 temperature (95 vol-% recovered), and boiling point ranges (from IBP to FBP, unless otherwise specified), reference is made to EN ISO 3405-2019. IBP is the temperature at the instant the first drop of condensate falls from the lower end of the condenser tube, and FBP is the maximum thermometer reading obtained during the test, usually occurring after the evaporation of all liquid from the bottom of the flask.

In the context of this disclosure, "sustainable" as in "sustainable hydrocarbon feed" refers to materials of renewable (biological) and/or circular origin.

Chemically, the renewable (herein also biological or biogenic) and non-renewable (such as petroleum) origin of any organic compound, including hydrocarbons, can be determined by suitable method for analysing the content of carbon from renewable sources e.g. DIN 51637:2014-02, ASTM D6866-2022, or EN 16640:2017. Said methods are based on the fact that carbon atoms of renewable or biological origin comprise a higher number of unstable radiocarbon (14C) atoms compared to carbon atoms of fossil origin. Therefore, it is possible to distinguish between carbon compounds derived from renewable or biological sources and carbon compounds derived from non-renewable (such as petroleum) sources by analysing the ratio of 12C and 14C isotopes. Thus, a particular ratio of said isotopes can be used as a "tag" to identify a renewable carbon compound and differentiate it from non-renewable carbon compounds. The isotope ratio does not change in the course of chemical reactions. Therefore, the isotope ratio can be used for identifying renewable carbon compounds and distinguishing them from non-renewable carbon compounds in (co-)feeds, streams, fractions, or compositions, or various blends thereof. Numerically, the biogenic carbon content can be expressed as the amount of biogenic carbon in the material as a weight percent of the total carbon (TC) in the material (in accordance with ASTM D6866-2022 or EN 16640:2017).

As used herein, the term circular in connection with content or materials such as (co-)feeds, streams, fractions, or compositions refers to content or material that is based on or contains reused and/or recycled non-biogenic carbon, but that may additionally contain biogenic carbon. Typical exemplary sources for reused and/or recycled non-biogenic carbon, possibly also containing at least some biogenic carbon, include reclaimed organic commodities, especially waste plastics, end of life tires, used lubricants, and/or municipal solid waste.

Renewable, circular, and petroleum content, materials, (co-)feeds, fractions, streams, or compositions are considered differing from one another based on their origin and impact on environmental issues. Therefore, they may be treated differently under legislation and regulatory framework. Typically, renewable, circular, and petroleum materials etc. are differentiated based on their origin and information thereof provided by the producer.

In the present disclosure, zeolite-related expressions like framework type, channel system, dimensionality, and similar, have the meanings as in the Atlas of zeolite framework types, 6^{th} revised edition, 2007. By hierarchical, or mesoporised zeolite is meant a zeolite wherein additional mesoporosity, or even macroporosity, has been introduced into the parent zeolite thus modifying its properties. The additional porosity enhances diffusion of reactants, e.g. bulkier molecules and/or with a longer chain such as isomerised hydrocarbon molecules, into the zeolite structure, and diffusion of product molecules out from the zeolite structure. Various approaches to produce hierarchical zeolites are known in the art. Regarding exemplary production methods of hierarchical zeolites and also characterisation methods of hierarchical zeolites and zeolites in general, reference may be made e.g. to a review by Hartmann et al., Hierarchically-Ordered Zeolites: A Critical Assessment and various approaches (Advanced Materials Interfaces, 2021, 8, 2001841).

In the present disclosure, the first and/or the second cracking catalyst may be in a ready-to-use state as such, or they may be treated in any customary way to adjust their properties, such as selectivity and/or activity, before or during start-up by subjecting for example to deactivation protocol, e.g. steaming, so as to obtain the ready-to-use fresh or ready-to-use regenerated cracking catalyst. As used herein, "cracking catalyst", and regenerated cracking catalyst, generally refer to the cracking catalyst in its ready-to-use state.

As used herein, "mean residence time" refers to the average time a cracking feed, excluding any diluent gas, is being subjected to the catalytic cracking reaction at the prevailing temperature and pressure, in the respective reaction zone.

In the present disclosure, the term "catalytically cracked stream" refers to the effluent from a step of catalytic cracking reaction, more specifically of the catalytic cracking reaction of step b) and/or step d) of the present process but excluding the catalyst and possibly formed coke, unless otherwise evident. This applies to all process configurations, whether using fixed or moving catalyst bed(s) such as fluidised catalyst bed.

A fraction "rich in propylene", means in the context of the present disclosure that the wt.-% amount of the propylene in the fraction, based on the total weight of the fraction, is higher than the wt.% amount of the propylene in the catalytically cracked stream wherefrom it has been separated, based on the total weight of the catalytically cracked stream. Preferably the wt.% amount of the propylene is higher than the wt.-% amount of any other single compound present in the fraction rich in propylene. In other words, the fraction rich in propylene comprises propylene as the most abundant compound. More preferably the fraction rich in propylene comprises more than 50 wt.% propylene, based on the total weight of the fraction rich in propylene. Meaning of "rich in" in connection with other fractions is as disclosed above for a fraction rich in propylene mutatis mutandis.

Sourcing of sustainable hydrocarbon feeds and/or of sustainable feeds for producing them is more complex, compared to conventional petroleum feeds, involving high compositional variation. Sustainable feeds are often sourced from many different sources, as their availability may vary, and the volumes of individual sources may be limited. Hence, flexibility i.e. the ability to convert varying feed compositions would be highly desired for a catalytic process aimed at processing sustainable hydrocarbon feeds. Changing the catalyst to a different one every time when the composition of the available sustainable hydrocarbon feed changes would be highly impractical and costly.

Furthermore, the light olefin yield, particularly the propylene yield, from a once-through single-stage catalytic cracking, is often modest i.a. due to limited conversion. Increasing the operating temperature may help to increase conversion but it cannot be increased indefinitely because at higher temperatures thermal cracking increases favouring ethylene formation over propylene. Recycling the unconverted portion of the cracking effluent back to the same cracking step may also help to increase conversion but it also increases the heterogeneity of the cracking feed, making it more challenging to optimise the cracking conditions for the majority of the feed molecules in the single cracking step.

As will become evident from the following, the two-stage catalytic cracking of the present method provides huge flexibility e.g. in terms of optimal utilisation of various fractions of the first and/or the second catalytically cracked stream, allowing to maximise yield of the desired product(s), particularly propylene, and/or alleviation of drawbacks such as undesired secondary reactions consuming the desired products, side product formation, coking, and/or undesirably fast catalyst deactivation, even when the variability of the composition and/or quality of the available sustainable hydrocarbon feed is high.

The present disclosure provides a method for producing propylene, the method comprising the following steps:
a) providing a sustainable hydrocarbon feed;
b) subjecting a first cracking feed comprising the sustainable hydrocarbon feed to catalytic cracking reaction in the presence of a first cracking catalyst comprising a first zeolite at a temperature within a range from 300 to 700 °C, preferably from 400 to 650 °C, to obtain a first catalytically cracked stream;
c) optionally separating from the first catalytically cracked stream at least a lighter fraction comprising at least C1-C3 hydrocarbons and a heavier fraction;
d) subjecting a second cracking feed comprising at least a portion, preferably the heavier fraction, of the first catalytically cracked stream to catalytic cracking reaction in the presence of a second cracking catalyst comprising a second zeolite at a temperature within a range from 350 to 750 °C, preferably from 450 to 700 °C, to obtain a second catalytically cracked stream; and
e) recovering from the second catalytically cracked stream, and optionally from the lighter fraction of the first catalytically cracked stream, at least a fraction rich in propylene;
wherein:
the first zeolite is selected from a group consisting of zeolites of a framework type having 10-membered ring as the largest ring-size (10 MR framework type), hierarchical zeolites of a 10 MR framework type, and zeolites of a framework type having 12-membered ring as the largest ring-size (12 MR framework type), preferably from hierarchical zeolites of a 10 MR framework type and zeolites of a 12 MR framework type,
the second zeolite is selected from a group consisting of zeolites of a 10 MR framework type, hierarchical zeolites of a 10 MR framework type, and zeolites of a 12 MR framework type, and
the second zeolite has a framework type that is different from the first zeolite unless both the first and the second zeolite are hierarchical zeolites of a 10 MR framework type,
with the proviso that when the first zeolite is a zeolite of a 10 MR framework type, the second zeolite is a hierarchical zeolite of a 10 MR framework type or a zeolite of a 12 MR framework type.

Surprisingly the present inventors found that an effective conversion of feeds having varying contents of different hydrocarbon species, particularly n-paraffins and isoparaffins, even bulky multibranched isoparaffins, is enabled by the two-stage catalytic cracking method of the present disclosure providing high propylene yields.

Conducting the re-cracking in a second catalytic cracking stage, instead of recycling to a single catalytic cracking stage, allows adjusting the conversion conditions e.g. based on the second cracking feed properties, to achieve optimal conditions for the second cracking catalyst, for optimal conversion level and/or for suppressing secondary reactions such as secondary hydrogen transfer reactions reducing propylene yields. For example, running the second cracking with shorter mean residence time and/or at higher temperature may help to reduce secondary hydrogen transfer reactions and hence yield to light olefins, particularly propylene. The two-stage approach also allows adjusting the conversion conditions in the first catalytic cracking, e.g. based on the sustainable hydrocarbon feed properties in view of the foreseen fluctuation thereof, to achieve optimal conditions for the first cracking catalyst, for optimal conversion level and/or for suppressing secondary reactions such as secondary hydrogen transfer reactions reducing propylene yields. This adjustment possibility, together with the selection of the first zeolite, enables to provide a beneficial composition to the first catalytically cracked stream, particularly to the heavier fraction thereof comprising C4+ hydrocarbons, preferably C5+ hydrocarbons. E.g. elevated olefins content in the heavier fraction may be desired, as it is easier to convert to the desired cracking products, particularly propylene, in the second catalytic cracking.

The two-stage approach provides further benefits also to the optional recycling of hydrocarbons having a carbon number of at least C4, preferably at least C5, separated from the second catalytically cracked stream: depending on the closer composition of the recycle stream and/or on the properties of the first and/or the second cracking catalyst, and the first and/or the second zeolite, respectively, the recycle stream may be introduced to the first or the second catalytic cracking, preferably to the second catalytic cracking, so as to optimise yield of the desired cracking products and/or to minimise coking and/or side product formation, or even split between both so as to e.g. divide the coking load between the first and the second catalyst. These benefits are not attainable by a one-stage catalytic cracking such as disclosed in WO22096781A1 or WO22096782A1 even when arranged with a recycle.

Using in the first catalytic cracking a first cracking catalyst comprising a first zeolite selected from zeolites of a framework type having 12-membered ring as the largest ring-size (12 MR framework type) or hierarchical zeolites of a framework type having 10-membered ring as the largest ring-size (10 MR framework type), provides enhanced accessibility and molecular traffic within the zeolite structure without significant mass transport limitations and active site accessibility constraints, even when the sustainable hydrocarbon feed has a high content of bulky isoparaffins. If a first zeolite having higher steric hindrance in its structure would be used, such as a non-hierarchical zeolite of 10 MR framework type, diffusion of the bulkier molecules would be limited and the reactions would occur more at acid sites located at or near the external surface, which might lead to reduced catalytic activity and conversion. To compensate this, when the first zeolite is a zeolite of a 10 MR framework type, the second zeolite is selected from hierarchical zeolites of a 10 MR framework type and zeolites of a 12 MR framework type.

The conversion and hence formation of the desired light olefins may be increased by increasing the mean residence time, but at the same time this would enhance the secondary reactions. Additionally, when the cracking reactions occur on the catalyst's external surfaces, secondary reactions tend to be favoured, particularly secondary hydrogen transfer reactions that are now less hindered by steric constraints. This leads to an increased formation of light paraffins and aromatics that can hardly be further converted to the desired light olefins in the second catalytic cracking step. Hence, the first zeolite is preferably selected from hierarchical zeolites of a 10 MR framework type and zeolites of a 12 MR framework type.

Additionally, using a second cracking catalyst comprising a second zeolite selected from zeolites of a 10 MR framework type, hierarchical zeolites of a 10 MR framework type, and zeolites of a 12 MR framework type provides accessibility and molecular traffic within the zeolite structure that is sufficient for the cracked molecules, without significant mass transport limitations and active site accessibility constraints. Selecting the second zeolite so that it has a framework type that is different from the first zeolite provides further opportunities for flexibility and/or optimisation. For example, the second zeolite may be selected optimally, e.g. based on the second cracking feed properties, to achieve optimal conditions for optimal conversion level, for suppressing secondary reactions such as secondary hydrogen transfer reactions reducing propylene yields and/or for optimal recycle stream properties. Surprisingly, in the conducted experiments it was observed, that excellent light olefins yields, particularly propylene yields, may also be attained when the first and the second zeolite are of the same framework type, particularly hierarchical zeolites of a 10 MR framework type, of which hierarchical IM-5 may be given as a preferred example. Without wishing to be bound to a theory it is believed that hierarchical zeolites of a 10 MR framework type exhibit a unique combination of sufficient space within the zeolite structure to avoid diffusion and mass transport limitations even when processing bulky isoparaffins, and sufficient confinement and steric hindrance within the zeolite structure to suppress secondary reactions also in the second stage where the feed contains already cracked molecules.

Preferably the present method comprises c) separating from the first catalytically cracked stream at least a lighter fraction comprising at least C1-C3 hydrocarbons and a heavier fraction, as this will significantly reduce secondary reactions of the desired products formed in the first catalytic cracking step, thereby enhancing their yield. Further, the light paraffins, particularly C1-C3 paraffins removed as part of the lighter fraction, are not effectively converted in the subsequent catalytic cracking to produce further light olefins but might even react with the light olefins formed in the subsequent catalytic cracking, thereby reducing their yield.

In certain preferred embodiments, the heavier fraction separated from the first catalytically cracked stream comprises hydrocarbons having a carbon number of at least C4, preferably at least C5, and optionally the heavier fraction is subjected to an aromatics removal and/or to a selective hydrogenation of diolefins to mono-olefins before incorporating into the second cracking feed. Typically, the first catalytically cracked stream contains some unconverted or uncracked components of the cracking feed, but also C4+ olefins, preferably C5+ olefins, which is particularly beneficial in the second catalytic cracking: one C4+ or C5+ olefin molecule may form 2 lighter olefins when cracked, while one C4+ or C5+ paraffin molecule may form 1 lighter olefin and 1 lighter saturated hydrocarbon. C4+, particularly C5+ olefins are also easier to crack than the paraffins having the same carbon number. Generally, C4-olefins are less convertible to propylene in the second catalytic cracking, or if recycled, and therefore the heavier fraction separated from the first catalytically cracked stream preferably comprises hydrocarbons having a carbon number of at least C5. Properties of the heavier fraction for use in the second cracking feed, and optionally for using a portion thereof as a recycle stream, may be further enhanced e.g. by subjecting to an aromatics removal and/or to a selective hydrogenation of diolefins to mono-olefins. This will reduce content of components increasing the risk of coking and/or side product formation in the subsequent catalytic cracking, and also reduce accumulation of recalcitrant components in the optional recycle loop(s).

The sustainable hydrocarbon feed used in the present method has typically a T5 temperature (5 vol-% recovered, EN ISO 3405-2019) at least 180 °C, preferably at least 190 °C, more preferably at least 200 °C, and a T95 temperature (EN ISO 3405-2019) at most 600 °C, preferably at most 550 °C, more preferably at most 500 °C. Preferred hydrocarbon feeds have both T5 and T95 temperatures (EN ISO 3405-2019), or even initial and final boiling points, within 180 °C - 650 °C, preferably within 190 °C - 600 °C, more preferably within 200 °C - 550 °C. Since the present method comprises two sequential catalytic cracking stages, the sustainable hydrocarbon feed may be selected flexibly within broad boiling characteristics, and also the first cracking stage may be operated flexibly within broad operating temperatures, not limited to operating in gas phase but allowing also operating in mixed liquid/gas phase. The second cracking stage ensures enhancement of the propylene yield in the overall process. Preferably the sustainable hydrocarbon feed has a difference between the T95 and T5 temperatures (EN ISO 3405-2019), preferably a difference between the initial and final boiling points, at most 300 °C, preferably at most 200 °C, more preferably at most 150 °C, even more preferably at most 100 °C, further more preferably at most 80 °C. Sustainable hydrocarbon feeds having relatively uniform composition in terms of boiling points are preferred as they may allow easier optimisation of the process conditions for the majority of the feed molecules, and in that way enhance formation of desired products and suppress formation of less desired products or side products. A well-defined boiling range is advantageous also for controlled and essentially complete evaporation of the feed, when operating in gas phase is desired.

Typically, the sustainable hydrocarbon feed comprises, based on the total weight of the sustainable hydrocarbon feed, at least 90 wt.%, preferably at least 95 wt.-%, more preferably at least 98 wt.% C10-C40 hydrocarbons. In certain preferred embodiments, the sustainable hydrocarbon feed comprises, based on the total weight of the sustainable hydrocarbon feed, at least 80 wt.%, preferably at least 85 wt.%, more preferably at least 90 wt.% C12-C30 hydrocarbons, and/or at least 75 wt.%, preferably at least 80 wt.%, more preferably at least 85 wt.% C12-C25 hydrocarbons. Sustainable hydrocarbon feeds having relatively uniform composition are preferred as they allow optimising the process conditions for the majority of the feed molecules, and in that way may enhance formation of desired products and suppress formation of undesired products.

In certain preferred embodiments, the sustainable hydrocarbon feed comprises, based on the total weight of the sustainable hydrocarbon feed, at least 50 wt.%, preferably at least 60 wt.%, more preferably at least 70 wt.% paraffins, even more preferably at least 90 wt.% paraffins, preferably having a carbon number of at least C12. In certain further preferred embodiments, the sustainable hydrocarbon feed comprises, based on the total weight of the sustainable hydrocarbon feed, at least 40 wt.%, preferably at least 50 wt.%, more preferably at least 60 wt.% paraffins having a carbon number of at least C14, and/or at least 25 wt.%, preferably at least 35 wt.%, more preferably at least 45 wt.% paraffins having a carbon number of at least C16. Paraffins are desired for the first cracking feed as they help to control or reduce coke formation in the catalytic cracking step, and paraffins, particularly long paraffins having a carbon number of at least C12 or more, tend to crack more easily compared to e.g. cyclic hydrocarbons or to shorter paraffins, making these feeds desired for the present catalytic cracking process.

In certain preferred embodiments, the sustainable hydrocarbon feed comprises, based on the total weight of the sustainable hydrocarbon feed, at least 20 wt.-%, preferably at least 40 wt.%, more preferably at least 60 wt.-%, even more preferably at least 80 wt.% isoparaffins. As explained in the foregoing, sourcing of the sustainable hydrocarbon feeds is complex, and they are often sourced from many different sources, e.g. due to availability limitations, causing high compositional variation. Hydrogenated vegetable and animal oil (HVO) processes are becoming more abundant, serving as one source for the sustainable hydrocarbon feed. However, HVO-processes may involve an isomerisation step, yielding a sustainable hydrocarbon stream having high content of isoparaffins, even highly bulky multibranched isoparaffins. The present method is particularly suited for feeds having varying content of isoparaffins of varying isomerisation degree, thanks to the two-stage catalytic cracking approach and careful selection of the zeolites, particularly the first zeolite, providing excellent flexibility regarding the feed composition. Hence, in certain preferred embodiments, the sustainable hydrocarbon feed comprises, based on the total weight of the sustainable hydrocarbon feed, at least 10 wt.-%, preferably at least 20 wt.-%, more preferably at least 30 wt.-%, even more preferably at least 40 wt.-% multibranched isoparaffins.

In certain preferred embodiments, the sustainable hydrocarbon feed comprises, based on the total weight of the sustainable hydrocarbon feed, 10 - 90 wt.-%, preferably 15 - 80 wt.-%, more preferably 20 - 70 wt.-%, even more preferably 30 - 60 wt.-% mono-olefins. Monoolefinic hydrocarbons may be desired for the first cracking feed as they tend to crack even more easily than e.g. paraffins of the same carbon number. On the other hand, olefins are also coke precursors, so their content in the sustainable hydrocarbon feed is preferably limited, especially when recycling of a portion of the first and/or the second catalytically cracked stream to the first catalytic cracking is desired, as that is foreseen to increase the olefins load in the first cracking feed. Hence in certain preferred embodiments, the sustainable hydrocarbon feed comprises, based on the total weight of the sustainable hydrocarbon feed, at most 40 wt.-%, preferably at most 30 wt.-%, more preferably at most 20 wt.-%, even more preferably at most 10 wt.-% mono-olefins.

Aromatics do not form or form only very little light olefins. Accordingly, they may accumulate in an optional recycle loop, unless specifically removed from the recycled stream, and tend to increase coke formation. Naphthenes also tend to decrease formation of light olefins, compared to paraffins, and naphthenes comprising a C6 ring are precursors for aromatics. Hence, in certain preferred embodiments, the sustainable hydrocarbon feed comprises, based on the total weight of the sustainable hydrocarbon feed, at most 15 wt.-%, preferably at most 10 wt.-%, more preferably at most 5 wt.-%, even more preferably at most 1 wt.-% aromatics, and/or at most 30 wt.-%, preferably at most 25 wt.-%, more preferably at most 10 wt.-%, even more preferably at most 5 wt.-% naphthenes.

Low oxygen content is desired for the sustainable hydrocarbon feed so as to avoid presence of oxygen-containing hydrocarbon species in the catalytically cracked stream, as oxygen-containing cracking products may be difficult to remove from the product fraction(s) containing the desired cracking product(s). Hence, in certain preferred embodiments, the sustainable hydrocarbon feed comprises, based on the total weight of the sustainable hydrocarbon feed, at most 3 wt.-%, preferably at most 2 wt.-%, more preferably at most 1 wt.-%, even more preferably at most 0.5 wt.-% oxygenated hydrocarbons, expressed as elemental oxygen (ASTMD5622-2017).

Sustainable hydrocarbon feeds suitable for the present method and having the characteristics as discussed above, may be prepared from various renewable i.e biomass feedstocks and/or circular feedstocks, using any suitable conversion method for the feedstock in question, particularly via hydroprocessing, such methods being well known in the art.

In certain preferred embodiments the sustainable hydrocarbon feed comprises at least one or more of a hydrotreated and optionally hydroisomerised biomass feedstock, a hydrotreated and optionally hydroisomerised waste plastic liquefaction oil, and/or a diene-depleted selectively hydrogenated waste plastic liquefaction oil. The hydrotreated and optionally hydroisomerised biomass feedstocks and waste plastic liquefaction oils are excellent sources of paraffins, and the diene-depleted selectively hydrogenated waste plastic liquefaction oil has elevated mono-olefin contents without the drawbacks of diolefins very easily causing side-reactions and -products. Mono-olefins may be seen as beneficial as they tend to crack more easily than paraffins of the same carbon number.

Exemplary biomass feedstocks include vegetable oils, animal fats, microbial oils, (ligno)cellulose-derived biocrude(s), or similar. Typical biomass feedstocks contain fatty acid(s), fatty acid glyceride(s), fatty acid alkyl ester(s), fatty alcohol(s), resin acid(s), resin ester(s), other oxygenated hydrocarbons, olefins, and/or cyclic hydrocarbons. In the present context the (ligno)cellulose-derived biocrude(s) refers to thermally such as hydrothermally or by pyrolysis, or catalytically such as thermo-catalytically liquefied (ligno)cellulosics, exemplary (ligno)cellulosics including e.g. woody biomass, agricultural (by)products, and/or (ligno)cellulosic industrial waste streams such as paper sludges and byproducts from food production.

Exemplary circular feedstocks include non-catalytically such as thermally (including hydrothermally and by pyrolysis) liquefied waste plastic and catalytically (including thermo-catalytically) liquefied waste plastic, herein generally referred to as waste plastic liquefaction oils. The waste plastic may encompass e.g. reclaimed waste plastics, end of life tires, and other similar materials.

If the biomass feedstocks and/or the waste plastic liquefaction oils include amounts or species of impurities that are not tolerated or preferred in the selected conversion, particularly in the hydrotreatment, hydroisomerisation and/or hydrogenation, their content may be reduced to acceptable limits using methods known in the art. Exemplary pretreatment methods suitable for the present disclosure comprise treating with mineral acids, degumming, treating with hydrogen, heat treatment, deodorizing, washing with water, treating with base, demetallation, distillation, removal of solids, bleaching, and any combinations thereof.

By hydrotreatment, sometimes also referred to as hydroprocessing, is meant herein a catalytic process of treating organic material by means of molecular hydrogen. The hydrotreating reactions may include removal of oxygen from oxygenated hydrocarbons as water (H2O), i.e. hydrodeoxygenation (HDO), sulphur from organic sulphur-containing compounds as dihydrogen sulphide (H2S), i.e. hydrodesulphurisation, (HDS), nitrogen from organic nitrogen-containing compounds as ammonia (NH3), i.e. hydrodenitrogenation (HDN), halogens, for example chlorine from organic chloride-containing compounds as hydrochloric acid (HCl), i.e. hydrodechlorination (HDCI), and/or metals by hydrodemetallization, and/or hydrogenation of olefinic bonds to saturated bonds and/or of aromatics to naphthenes. Depending e.g. on the composition of the hydrotreatment feed, different reactions may occur and/or prevail in the hydrotreatment. Hydrotreatment may also involve certain side reactions, such as cracking reactions or ring-opening. Hydrotreatment is a preferred conversion method for providing the sustainable hydrocarbon feed because it is capable of converting hydrotreatment feeds of varying compositions to highly paraffinic materials, and at the same time reduce content of heteroatoms, metals, olefins, aromatics and/or other impurities in the biomass feedstocks and/or in the circular feedstocks, particularly the waste plastic liquefaction oils.

Hence, in certain particularly preferred embodiments, providing the sustainable hydrocarbon feed of step a) of the present method comprises:
a1) providing a biomass feedstock and/or a waste plastic liquefaction oil,
a2) subjecting the biomass feedstock and/or the waste plastic liquefaction oil, optionally mixed with a hydrocarbon liquid diluent, to hydrotreating reaction(s) to produce a hydrotreated stream,
a3) subjecting the hydrotreated stream to a gas-liquid separation to produce at least a gaseous stream and a hydrotreated liquid stream,
a4) optionally subjecting at least a part of the hydrotreated liquid stream to a hydroisomerization step to produce a hydroisomerized stream;
a5) separating the sustainable hydrocarbon feed from the hydrotreated liquid stream, from the hydroisomerized stream and/or from a combination thereof, by dividing, stabilizing and/or fractionating.

The optional hydrocarbon liquid diluent may help to control reactor exotherm, i.e. heat released by the hydrotreating reaction(s) and/or to increase solubility of hydrogen in the hydrotreating reaction mixture. Exemplary hydrocarbon liquid diluents include product recycle such as a portion of the hydrotreated liquid stream, the hydroisomerized stream or a combination thereof, and/or various petroleum streams such as petroleum distillate(s) and/or petroleum (hydro)crackate(s).

The hydrotreating reaction(s) are typically carried out at conditions comprising at least one or more of:
a temperature in the range from 120 °C to 500 °C,
a pressure in the range from 10 bar to 200 bar,
a WHSV in the range from 0.1 h⁻¹ to 10 h⁻¹,
a H₂ flow of from 50 to 2000 N-L H₂/L feed, and/or
a hydrotreating catalyst, preferably a sulphided hydrotreating catalyst, comprising at least one or more metals from Group VIII of the Periodic Table and/or from Group VIB of the Periodic Table, preferably at least one or more of Ni, Mo, W, and/or Co, even more preferably at least one or more of Ni and/or Co and Mo and/or W, such as NiMo, CoMo, NiCoMo, NiW, and/or NiMoW, preferably on a support, more preferably on alumina.

Hydrotreating conditions effective for HDO may reduce the oxygen content of oxygen containing feedstocks to less than 1 wt.-%, such as to less than 0.5 wt.-% or to less than 0.2 wt.-%, based on the total weight of the hydrotreated liquid stream or the sustainable hydrocarbon feed separated therefrom.

In certain embodiments, the sustainable hydrocarbon feed comprises a hydrotreated waste plastic liquefaction oil. The hydrotreatment may be as carried out at conditions specified, e.g., in FI130219B.

In certain embodiments, the sustainable hydrocarbon feed comprises a diene-depleted selectively hydrogenated waste plastic liquefaction oil. The selective diolefin hydrogenation may be conducted e.g. as disclosed in WO2022/144490.

The optional hydroisomerisation step may be conducted using well known technologies, such as those depicted in FI100248B, EP1741768A1, EP2155838B1, FI129220B, EP1396531 A2, or WO9856876A1, to reach the targeted isomerisation degree. The severity of the hydroisomerisation and thereby the degree of isomerisation may be increased e.g. by decreasing WHSV, increasing temperature, and/or increasing pressure.

There may be further steps included either combined with the hydrotreatment, hydroisomerization and/or hydrogenation, or thereafter. These may comprise e.g. gas-liquid separation, hydropolishing, dearomatizing, just to name a few. Typically, such additional process steps aim at better control of desired properties of the hydrotreated, hydroisomerized or hydrogenated stream.

Surprisingly, the present inventors found that by conducting the catalytic cracking in two stages wherein the first cracking catalyst comprises a first zeolite and the second cracking catalyst comprises a second zeolite, and wherein the first zeolite is selected from a group consisting of zeolites of a framework type having 10-membered ring as the largest ring-size (10 MR framework type), hierarchical zeolites of a 10 MR framework type, and zeolites of a framework type having 12-membered ring as the largest ring-size (12 MR framework type), preferably from hierarchical zeolites of a 10 MR framework type and zeolites of a 12 MR framework type; the second zeolite is selected from a group consisting of zeolites of a 10 MR framework type, hierarchical zeolites of a 10 MR framework type, and zeolites of a 12 MR framework type; and the second zeolite has a framework type that is different from the first zeolite unless both the first and the second zeolite are hierarchical zeolites of a 10 MR framework type, with the proviso that when the first zeolite is a zeolite of a 10 MR framework type, the second zeolite is a hierarchical zeolite of a 10 MR framework type or a zeolite of a 12 MR framework type, it is possible to convert sustainable hydrocarbon feeds to propylene with excellent conversion and high flexibility especially in view of the sustainable hydrocarbon feed properties, compared to the prior art catalytic cracking processes. E.g., ZSM-5 which is a typical cracking catalyst, is of 10 MR framework type, exhibiting higher steric hindrance in its structure, and therefore facilitating more of the cracking reactions on its external surfaces, where the occurrence of secondary hydrogen transfer reactions on the other hand is less hindered by steric constraints. However, in the conducted experiments it was observed that when the first zeolite is a zeolite of a 10 MR framework type, its limitations may be compensated to some extent by selecting the second zeolite from hierarchical zeolites of a 10 MR framework type or zeolites of a 12 MR framework type. Preferably the first zeolite is selected from hierarchical zeolites of a 10 MR framework type and zeolites of a 12 MR framework type as these exhibit enhanced access to acid sites within their channels, where secondary hydrogen transfer reactions are more sterically restricted. The specified selection of second zeolites provides accessibility and molecular traffic within the zeolite structure that is sufficient for the cracked molecules, without significant mass transport limitations and active site accessibility constraints. However, when the first zeolite is a zeolite of a 10 MR framework type, the second zeolite shall be selected from hierarchical zeolites of a 10 MR framework type or zeolites of a 12 MR framework type to ensure that the elevated content of unconverted first cracking feed molecules present in the second cracking feed get converted, even when these molecules are bulky. As explained in the foregoing, by selecting the second zeolite so that it has a framework type that is different from the first zeolite provides further opportunities for flexibility and/or optimisation, while excellent light olefins yields, particularly propylene yields, may also be attained when the first and the second zeolite are of the same kind, particularly hierarchical zeolites of a 10 MR framework type, of which hierarchical IM-5 may be given as a preferred example.

The benefits may be further enhanced and/or drawbacks reduced by carefully selecting the first zeolite and the second zeolite within the above-specified zeolite types.

Hence, in certain embodiments, the second zeolite is selected from zeolites of a 10 MR framework type, hierarchical zeolites of a 10 MR framework type, and zeolites of a 12 MR framework type, having largest pore diameter smaller than the largest pore diameter in the framework type of the first zeolite, preferably from zeolites of a 10 MR framework type and hierarchical zeolites of a 10 MR framework type. These embodiments may provide within the second zeolite structure a good combination of sufficient diffusion and mass transport as well as sufficient confinement and steric hindrance to suppress secondary reactions, including secondary hydrogen transfer reactions, that would consume the formed desired products and form e.g. light paraffins, aromatics and coke, in the second stage where the feed contains already cracked molecules and olefins. Both accessibility constraints and too low confinement effect may lead to reduced activity, necessitating longer residence times, that in turn enhance secondary reactions, particularly secondary hydrogen transfer reactions.

In certain preferred embodiments, the second zeolite is selected so that the largest pore diameter in the framework type is smaller than the largest pore diameter in the framework type of the first zeolite. These embodiments may provide a further enhanced confinement effect for the cracked molecules in the second zeolite, as discussed above.

In certain preferred embodiments, the first zeolite is a zeolite of a 10 MR framework type, and the second zeolite is a zeolite of a 12 MR framework type. These embodiments may further help to compensate for the mass transport limitations within the first zeolite having smaller pores that the sustainable hydrocarbon feed molecules encounter.

In certain preferred embodiments, the first zeolite is of a framework type wherein the channel system dimensionality is higher compared to the channel system dimensionality of the second zeolite. These embodiments may provide a further enhanced confinement effect for the cracked molecules in the second zeolite and enhanced mass transport for the sustainable hydrocarbon feed molecules in the first zeolite.

In certain preferred embodiments, the first and the second zeolite are hierarchical zeolites of a 10 MR framework type, preferably hierarchical zeolites of the same 10 MR framework type, more preferably hierarchical zeolites of the same 10 MR framework type and mesoporosity, even further preferably hierarchical IM-5 of the same mesoporosity. The benefits of these embodiments are as discussed in the foregoing and shown in the experiments.

In certain preferred embodiments, the first and the second zeolite are of the same framework type, but the second zeolite has a lower Si/AI molar ratio compared to the first zeolite. A lower Si/AI molar ratio reflects higher acid density, and generally higher activity. By selecting a second zeolite with a lower Si/AI molar ratio, i.e. higher acid density and activity compared to the first zeolite of the same framework type, the second catalytic cracking may be run using shorter mean residence time. The second catalytic cracking feed has a higher olefin content to start with, so that a shorter mean residence time would help to suppress undesired secondary reactions, while the higher activity ensures sufficient conversion level.

In certain preferred embodiments, the first zeolite and the second zeolite have a Si/AI molar ratio, selected independently from each other, within a range from 5 to 250, preferably from 5 to 150, more preferably from 10 to 100. The Si/AI molar ratio of the first and/or the second zeolite may be selected or adjusted preferably within the specified ranges, providing acidity that is high enough to ensure good cracking activity and/or sufficient resistance to deactivation during the cracking, and low enough to suppress side reactions such as formation of aromatic compounds to avoid accumulation of carbonaceous deposits on acid sites and blockage of pore channels, thereby helping to maintain the catalytic activity. Adjusting the Si/AI molar ratio may be conducted with any known technique, including pre-modification and/or post-modification techniques. For example, a zeolite having a framework type with Si/AI ratio close to a desired value may be subjected to dealumination and/or desilication treatment to further adjust the properties of the zeolite.

In certain preferred embodiments, the first zeolite is of a framework type MFI (largest ring-size 10MR, 3D channel system, e.g. ZSM-5), IMF (largest ring-size 10MR, 2D channel system, e.g. IM-5), ITH (largest ring-size 10MR, 3D channel system, e.g. ITQ-13), FER (largest ring-size 10MR, 2D channel system, e.g. ferrierite), MWW (largest ring-size 10MR, 2D channel system, e.g. MCM-22), TON (largest ring-size 10MR, 1D channel system, e.g. ZSM-22), MTT (largest ring-size 10MR, 1D channel system, e.g. ZSM-23), MTW (largest ring-size 12MR, 1D channel system, e.g. ZSM-12), BEA (largest ring-size 12MR, 3D channel system, e.g. beta), FAU (largest ring-size 12MR, 3D channel system, e.g. USY), MSE (largest ring-size 12MR, 3D channel system, e.g. MCM-68) or IWV (largest ring-size 12MR, 2D channel system, e.g. ITQ-27), preferably of a framework type MFI, IMF, MTW, BEA or FAU. In certain preferred embodiments the second zeolite is of a framework type MFI, IMF, ITH, FER, MWW, TON, MTT, MTW, BEA, FAU, MSE, or IWV, preferably of a framework type MFI, IMF, MTW, BEA, or FAU.

In certain particularly preferred embodiments, the first zeolite is ZSM-12 and the second zeolite is ZSM-5, or the first zeolite is beta zeolite and the second zeolite is ZSM-5, or the first zeolite is ITQ-27 and the second zeolite is ZSM-5, or the first zeolite is MCM-68 and the second zeolite is ZSM-5, or the first zeolite is hierarchical IM-5 and the second zeolite is ZSM-5, or the first zeolite is hierarchical IM-5 and the second zeolite is hierarchical IM-5. These provide good accessibility in the first zeolite even for bulky multibranched isoparaffins and sufficient confinement effect in the second zeolite for steric hindrance to suppress side reactions.

In certain embodiments, the first zeolite and/or the second zeolite may have a framework type comprising crystals having a submicron crystal size. Reducing the size of zeolite crystals shortens diffusional paths and it is expected that the produced chemicals will diffuse more quickly outside of the zeolitic structure, thereby potentially minimizing the occurrence of secondary bimolecular reaction mechanisms, such as secondary hydrogen transfer. Furthermore, these materials with smaller crystallites generally exhibit an increased external surface area and consequently a higher proportion of active sites close to the crystal surface, leading to enhanced access to these active sites for reactants.

Typically, the first and the second cracking catalyst further comprise at least one or more supports. In this context the expression support comprises any binders and/or fillers. The support may comprise amorphous silica, alumina, amorphous silica-alumina, zirconia, a clay material, such as kaolin or bentonite, that may be further thermally or chemically treated. Depending on the type of reactor used in the catalytic cracking of step b) or d), the support needs to meet different specifications related to mechanical strength and formability. For example, a catalyst with a silica support deactivates slowly which has an advantage when the cracking reaction is performed in a fixed or moving bed reactor. Dopants such as zinc or gallium may also be included, but preferably the cracking catalyst does not contain dopants. In some embodiments the first or the second cracking catalyst may also comprise minor amounts of other components, such as conventional catalyst additives, and/or other zeolites than the first or the second zeolite, respectively.

The catalytic cracking step b) of the present disclosure is performed at a temperature in a range from 300 to 700 °C, preferably in a range from 400 to 650 °C, and the catalytic cracking step d) of the present disclosure is performed at a temperature in a range from 350 to 750 °C, preferably in a range from 450 to 700 °C. Typically, a mean residence time of the first cracking feed in step b), and/or a mean residence time of the second cracking feed in step d), may vary from 0.1 s to 150 s, preferably from 0.2 s to 100 s such as from 0.2 s to 75 s, more preferably from 0.2 s to 50 s or even from 0.2 s to 35 s. Generally, the shorter the mean residence time the less there is time for the desired cracking products to be consumed by secondary reactions. Also coke precursors, including olefins, polyolefins, saturated and unsaturated naphthenes, and aromatics, that are highly active hydrogen donor molecules, have less time to undergo e.g. alkylation, condensation, and polymerization reactions. The desired cracking products and coke precursors are foreseen more abundant in the second cracking feed. Hence, in certain preferred embodiments, the mean residence time of the second cracking feed in step d) is shorter than the mean residence time of the first cracking feed in step b).

Typically, the total pressure at catalytic cracking step b) and catalytic cracking step d) is selected, independently from each other, within a range from 50 kPa to 150 kPa (absolute), preferably from 80 kPa to 120 kPa (absolute), more preferably step b) and step c) are conducted at about atmospheric pressure. The present process is preferably conducted at a pressure not significantly higher than ambient pressure so as to decrease risk of the formed light olefins, such as the desired propylene, to react further. Furthermore, typically when the reaction is performed above atmospheric pressure the yields of the light olefins are decreased compared to when the reaction is performed at atmospheric pressure.

Since the present process is for producing light olefins, particularly propylene, the catalytic cracking reactions are performed without added hydrogen. However, a minor amount of hydrogen may result from the reactions taking place during cracking. Additionally, a minor amount of hydrogen may be carried over as part of the sustainable hydrocarbon feed, as part of the first catalytically cracked stream, and/or as part of the second catalytically cracked stream if recycling a portion thereof to the first and/or second cracking feed.

In preferred embodiments, the first and/or the second cracking feed comprises, based on the total weight of the cracking feed, from 0.5 wt.-% to 50 wt.-%, preferably from 0.5 wt.-% to 30 wt.%, more preferably from 1 wt.-% to 20 wt.-% a diluent gas. In this context by the diluent gas it is meant steam or compounds that are gaseous at NTP. Typically, the diluent gas is non-reactive or essentially non-reactive under the conditions that prevail in the catalytic cracking step. Preferably the diluent gas comprises at least one or more of steam, methane, ethane, propane, nitrogen, carbon monoxide, carbon dioxide, and/or saturated C1-C4 hydrocarbons separated from the first and/or the second catalytically cracked stream. By incorporating a diluent gas to the cracking feed, the partial pressure of the hydrocarbons in the cracking feed may be decreased, thus favouring formation of light olefins and suppressing formation of light paraffins. This enhanced olefinicity is attributed to the reduced occurrence of secondary hydrogen transfer reactions under diluted hydrocarbon conditions. These reactions are more sensitive to changes in hydrocarbon partial pressure compared to the unimolecular cracking reactions that primarily drive conversion. Additionally, operating at low hydrocarbon partial pressures may facilitate the dispersion and vaporization of the cracking feeds at the reactor inlet, potentially enhancing diffusion efficiency within the porous catalyst network. This may result in a shorter contact time between the feed/products and the catalyst, leading to a proportional reduction in thermal cracking and secondary reactions. As a result, dry gas and coke yields would decrease, further improving selectivity towards more desired products. The lower hydrocarbon partial pressures may reduce cracking reaction rates, but the achieved reduced coke deposition and catalyst deactivation are foreseen to partially offset the decline in conversion.

While WO2022096781 and WO2022096782 were directed to one-stage catalytic cracking with optional recycle to enhance product yields, the present two-stage catalytic cracking is more advantageous regarding optional recycling of a fraction separated from the second catalytically cracked stream: depending on the composition of the recycle stream and/or on the properties of the first and/or the second cracking catalyst, and the first and/or the second zeolite, respectively, the recycle stream may be introduced to the first or to the second catalytic cracking so as to optimise yield of the desired cracking products and/or to minimise coking and/or side product formation, or it may even be split between both so as to e.g. divide the coking load between the first and the second catalyst. This may not be attained by recycling in a one-stage catalytic cracking process. Hence, in certain particularly preferred embodiments, the method of the present disclosure comprises separating from the second catalytically cracked stream a fraction of hydrocarbons having a carbon number of at least C4, preferably at least C5, and recycling at least a portion thereof to the first and/or the second cracking feed, preferably to the second cracking feed, optionally after subjecting to an aromatics removal and/or to a selective hydrogenation of diolefins to mono-olefins. The aromatics removal may be conducted e.g. by extractive distillation or solvent extraction. Selective hydrogenation of diolefins may be conducted as disclosed e.g. in WO 2022/144490.

In certain preferred embodiments, the first cracking feed comprises, based on the total weight of the first cracking feed, at least 40 wt.-%, preferably at least 50 wt.-%, more preferably at least 60 wt.-%, even more preferably at least 90 wt.-% the sustainable hydrocarbon feed, or may even consist essentially thereof. Preferably in these embodiments, the remaining of the first cracking feed consists of a diluent gas and/or a recycle stream of hydrocarbons having a carbon number of at least C4, preferably at least C5, separated from the first and/or the second catalytically cracked stream.

In certain embodiments, the first cracking feed may also comprise at least one or more fossil crude oil distillate and/or fossil crude oil (hydro)crackate as a co-feed, preferably of similar distillation characteristics as the sustainable hydrocarbon feed.

In certain preferred embodiments, the sum amount of the at least a portion of the first catalytically cracked stream, and a diluent gas and/or a recycle stream of hydrocarbons having a carbon number of at least C4, preferably at least C5, separated from the first and/or the second catalytically cracked stream is at least 80 wt.-%, preferably at least 90 wt.-%, more preferably at least 95 wt.-% or 100 wt-%, based on the total weight of the second cracking feed.

In certain embodiments, the present method further comprises vaporizing the first and/or the second cracking feed before subjecting to the catalytic cracking reaction. In this way it is possible to ensure that the cracking feed is in gas phase when subjected to the catalytic cracking reaction, allowing higher feed rates with less pressure drop, less limitations to the mass transport and better mixing characteristics in general, compared to processes where the feed is not fully or at all in gas phase.

In certain embodiments, in step b) the first cracking catalyst and/or in step d) the second cracking catalyst is arranged at least in one or more catalyst bed(s) in one or more reactor(s), preferably at least in one or more fluidised catalyst bed reactor(s) and/or fixed catalyst bed reactor(s). Process configurations using fixed catalyst bed(s) are more simple and less expensive, while process configurations using moving catalyst, such as fluidised, ebullated, or slurry catalysts, are more flexible regarding coke-formation and catalyst regeneration.

The catalyst regeneration may be conducted in any conventional manner, e.g. continuously or batch-wise. Typically, in a fixed bed reactor, the cracking catalyst is not regenerated during the catalytic cracking, but in separate regeneration cycles during which catalytic cracking cannot be performed in the fixed bed reactor being regenerated. When using fixed catalyst beds, in order to reduce or avoid down-time, a swing reactor concept may be utilised wherein one reactor is removed from service for regeneration and a freshly regenerated reactor is simultaneously returned to service. When using moving solid catalyst reactors, it is possible to renew the cracking catalyst, or to regenerate it during the catalytic cracking, e.g. in a separate coke burning reactor. In embodiments where the operating temperature of the respective cracking reactor is close to typical catalyst regeneration temperatures, the regenerated catalyst may not require significant cooling after the regeneration, before re-introducing to the catalytic cracking reactor. This may provide shorter catalyst regeneration cycle time.

The second catalytically cracked stream may be separated into different fractions using known separation methods. For example, the second catalytically cracked stream may be removed from the used reactor via an overhead line, cooled and sent to fractionation, such as a fractionator tower, for recovering of the various cracking products. The separation of the fractions and recovery of the various cracking products may be conducted in several steps.

The separation and/or recovery, e.g. in step c) and/or step e), may comprise for example at least one or more of dividing, stabilising, fractionating, distilling, evaporating, flash-separating, membrane separating, extracting, using extractive-distillation, using chromatography, using molecular sieve adsorbents, using thermal diffusion, complex forming, crystallising, preferably at least dividing, stabilizing and/or fractionating. The separation and/or recovery may comprise multiple unit operations in parallel and/or succession. Usable processing units for separation of the fractions and/or recovery of the cracking products may include de-methanizers, de-ethanizers, de-propanizers, de-butanizers, propane-propylene splitters, and/or ethane-ethylene splitters, just to name a few.

According to the method at least a fraction rich in propylene is recovered from the second catalytically cracked stream, and optionally from the lighter fraction of the first catalytically cracked stream. One of the benefits of the present method is a high propylene content in the C3 fraction separated in the present process, herein also referred to as C3 olefinicity. Hence, a separated C3 fraction may have a propylene content of a chemical grade propylene already as such, and require less effort, less expensive equipment, and less energy to be refined to polymer grade purity compared to C3 fractions having lower propylene content.

In certain preferred embodiments, step e) further comprises recovering from the second catalytically cracked stream, and optionally from the lighter fraction of the first catalytically cracked stream, a fraction rich in ethylene, a fraction rich in one or more of C4 mono-olefins, and/or a fraction rich in saturated C1-C4 hydrocarbons; and/or recovering from the second catalytically cracked stream a fraction of hydrocarbons having a carbon number of at least C4, preferably at least C5, and/or a fraction rich in aromatics.

The present method provides a high ethylene content in the recovered C2 fraction, herein also referred to as C2 olefinicity. Hence, the recovered C2 fraction may be usable even as such, and require less effort, less expensive equipment, and less energy to be refined to chemical grade or even polymer grade purity compared to C2 fractions having lower ethylene content.

The present method may further comprise recovering from the second catalytically cracked stream, and optionally from the lighter fraction of the first catalytically cracked stream, a fraction rich in one or more of C4 mono olefin(s), that may be useful e.g. as (co)monomers as such or as further derivatised, in the production of butyl rubber by polymerisation, isooctane by alkylation, diisobutylene by dimerization, and methyl and ethyl tert-butyl ethers by reacting with methanol or ethanol, respectively.

Overall yield of propylene may be increased by cracking at least part the fraction of hydrocarbons having a carbon number of at least C4, preferably at least C5 and/or at least part of the fraction rich in saturated C1-C4, preferably saturated C2-C4 hydrocarbons. Hence, in certain further preferred embodiments the present method includes subjecting at least a portion of the fraction of hydrocarbons having a carbon number of at least C4, preferably at least C5, recovered from the second catalytically cracked stream, after optionally subjecting to aromatics removal e.g. by extractive distillation or solvent extraction and/or to a selective hydrogenation of diolefins to mono olefins, to a further cracking reaction to produce a further cracked stream, wherein the further cracking reaction is a catalytic cracking reaction in the presence of a cracking catalyst; and/or at least a portion of the fraction rich in saturated C1-C4, preferably saturated C2-C4 hydrocarbons, recovered from the second catalytically cracked stream to a further cracking reaction to produce a further cracked stream, wherein the further cracking is thermal cracking, preferably steam cracking; followed by separating from the further cracked stream(s) at least a fraction rich in propylene. When coupling the two-stage catalytic process with a small steam cracking unit, the steam cracking yields would be highly dependent of the processed feedstock but typically providing 80 wt.-% of ethylene, 2 wt.-% of propylene and 3 wt.-% of butenes from ethane, 45 wt.-% of ethylene, 15 wt.-% of propylene and 3 wt.-% of butenes for propane and 37 wt.-% of ethylene, 18 wt.-% of propylene and 8 wt.-% of butenes for butanes, i.e. providing further propylene although with low propylene to ethylene ratio.

In certain embodiments, the method comprises separating from the first and/or second catalytically cracked stream a fraction rich in saturated C1-C4 hydrocarbons, and recycling at least a part thereof to the first and/or second cracking feed. The fraction rich in saturated C1-C4 hydrocarbons may act as a diluent gas, decreasing partial pressure of the hydrocarbons in the cracking feed, thus reducing occurrence of secondary hydrogen transfer reactions and promoting formation of light olefins, including propylene.

Regarding the cracking products typically present in the first and/or second catalytically cracked stream in varying amounts, the C2-C4 paraffins, may also be used e.g. as a component in a catalytic dehydrogenation feed, to further produce light olefins. Methane, ethane, propane and/or C4-paraffins may also be used as a component in a steam-reforming feed to produce H₂, and/or in a fuel gas composition. A fraction rich in aromatics recoverable from the second catalytically cracked stream may find use in solvents, specialty chemicals production and/or production of aromatic monomers and polymers.

### Schematic presentation of the method

Fig. 1 schematically shows an example embodiment of the method according to the present disclosure, illustrating also certain preferred although optional features. The method shown in Figure 1 comprises providing a sustainable hydrocarbon feed **A,** for example by hydrotreating **10** a biomass feedstock and/or a waste plastic liquefaction oil, and subjecting a first cracking feed comprising the sustainable hydrocarbon feed **A** to catalytic cracking reaction **20** in the presence of a first cracking catalyst comprising a first zeolite to obtain a first catalytically cracked stream **B.** At least a lighter fraction **B_{L}** comprising at least C1-C3 hydrocarbons and a heavier fraction **B_{H}** may be separated **25** from the first catalytically cracked stream **B,** and the heavier fraction **B_{H}** may be subjected to separation of aromatic hydrocarbons **G** and/or to a selective hydrogenation of diolefins to mono-olefins (not shown in Figure 1). A second cracking feed comprising at least the heavier fraction **B_{H}** of the first catalytically cracked stream **B** is subjected to catalytic cracking reaction **30** in the presence of a second cracking catalyst comprising a second zeolite to obtain a second catalytically cracked stream **C.** In Figure 1, at least a lighter fraction **C_{L}** comprising at least C1-C3 hydrocarbons and a heavier fraction **C_{H}** are separated **40** from the first catalytically cracked stream **C**, and from the lighter fraction **C_{L}** of the second catalytically cracked stream **C**, optionally as combined with the lighter fraction of the first catalytically cracked stream **B_{L},** at least a fraction rich in propylene **D** is recovered.

Figure 2 schematically shows an example embodiment of the method according to the present disclosure, illustrating also certain preferred although optional features. The method shown in Figure 2 comprises providing a sustainable hydrocarbon feed **A,** for example by hydrotreating **10** a biomass feedstock and/or a waste plastic liquefaction oil, and subjecting a first cracking feed comprising the sustainable hydrocarbon feed **A** and optionally a diluent gas **H** to catalytic cracking reaction **20** in the presence of a first cracking catalyst comprising a first zeolite to obtain a first catalytically cracked stream **B.** A second cracking feed comprising at least a portion of the first catalytically cracked stream **B** and optionally a diluent gas **H** is subjected to catalytic cracking reaction **30** in the presence of a second cracking catalyst comprising a second zeolite to obtain a second catalytically cracked stream **C.** In Figure 2 at least a fraction rich in propylene **D** and a propylene-depleted stream **E** are recovered **40** from the second catalytically cracked stream **C.** At least a portion of the propylene-depleted stream **E** may be recycled as a recycle stream **F** to the first and/or the second cracking feed. Prior to the recycling, the stream **F** is preferably subjected to separation of aromatic hydrocarbons **G** and/or to a selective hydrogenation of diolefins to mono-olefins (not shown in Figure 2).

### EXPERIMENTAL

### Sustainable hydrocarbon feeds

Three different sustainable hydrocarbon feeds were prepared. R1 and R2 are renewable hydrocarbon feeds prepared by catalytic hydrodeoxygenation of conventionally purified glyceridic feed of animal fat/vegetable oil origin mixed with a hydrotreated liquid stream (product recycle) as diluent, followed by gas-liquid separation. Hydrocarbon feed R2 was additionally hydroisomerized followed by gas-liquid separation and stabilisation. C1 was a circular hydrocarbon feed prepared by catalytic hydrotreatment of conventionally purified liquefied waste plastic (obtained by thermal liquefaction/pyrolysis), followed by gas-liquid separation and fractionation. The characteristics of the thus obtained sustainable hydrocarbon feeds R1, R2 and C1 were then analysed and are reported in Table 1.

**Table 1. Characteristics of the hydrotreated sustainable hydrocarbon feeds. Chemical composition was determined by GCxGC-FID/GCxGC-MS.**

| Characteristic | R1 | R2 | C1 |
|---|---|---|---|
| Density at 15°C, kg/m³ (ENISO12185:1996) | 782 | 780 | n.a. |
| Distillation (ENISO3405:2019) | | | |
| IBP, °C (initial boiling point) | 268 | 203 | 200 |
| T5, °C (5 vol-% recovered) | 285 | 245 | |
| T10, °C (10 vol-% recovered) | 287 | 257 | |
| T50, °C (50 vol-% recovered) | 294 | 281 | |
| T90, °C (90 vol-% recovered) | 304 | 292 | |
| T95, °C (95 vol-% recovered) | 307 | 296 | |
| FBP, °C (final boiling point) | 320 | 305 | 350 |
| N-paraffins, wt.-% | 97 | 7 | 46 |
| Isoparaffins, wt.-% | 3 | 93 | 26 |
| Multibranched isoparaffins, wt.-% | <1 | 50 | 16 |
| Total paraffins, wt.-% | 100 | 100 | 72 |
| ≥C12 paraffins, wt.-% | 100 | 96 | 69 |
| ≥C16 paraffins, wt.-% | 90 | 81 | 55 |
| Hydrocarbons, wt.-% | 100 | 100 | 100 |
| C10-C40, wt.-% | 100 | 99 | 100 |
| C12-C25, wt.-% | 99 | 95 | 95 |
| ≥C30, wt.-% | 0 | 0 | 0 |
| Naphthenes, wt.-% | <1 | <1 | 22 |
| Aromatics, wt.-% | | | 6 |

As can be seen from Table 1, all the hydrocarbon feeds R1, R2 and C1 were highly paraffinic, had ≥C16 paraffins as the major constituent and very low aromatics content, and were essentially free from oxygen and olefins. All three feeds had desired characteristics for use in the present method. The renewable hydrocarbon feeds R1 and R2 even had very low total content of cyclic hydrocarbons (summed amount of aromatics and naphthenes). Hydrocarbon feeds R1 and R2 were used in the catalytic cracking experiments as described in the following.

### Catalysts

MFI type zeolite, such as ZSM-5, a highly siliceous aluminosilicate microporous zeolite with an intersecting and three-dimensional channel system is a shape selective catalyst characterized by a 10-membered ring (10-MR) pore structure having straight channels with pore openings of 5.1 × 5.5 Å connected by sinusoidal channels of 5.3 × 5.6 Å and intersection cavities of around 9 Å. Three different ZSM-5 catalysts having the following textural properties were used in the experiments:
ZSM-5 (31) having Si/AI molar ratio 31 measured through ICP-OES analysis; BET area 389 m²/g; micropore area 343 m²/g; external area 46 m²/g; micropore volume 0.17 cm³/g; mesopore volume 0.07 cm³/g determined through nitrogen physisorption using BET and t-plot method; crystal size 0.3-0.7 microns estimated through SEM analysis.
ZSM-5 (125) having Si/AI molar ratio 125; BET area 368 m²/g; micropore area 325 m²/g; external area 42 m²/g; micropore volume 0.16 cm³/g; mesopore volume 0.11 cm³/g; crystal size 0.5-1.0 microns.
ZSM-5 (400) having Si/AI molar ratio 400; BET area 417 m²/g; micropore area 378 m²/g; external area 39 m²/g; micropore volume 0.18 cm³/g; mesopore volume 0.05 cm³/g; crystal size 0.2-0.3 microns.

MTW type zeolite such as ZSM-12, a silica rich microporous zeolite with unidimensional 12-membered ring channel system and pore openings of 5.7 × 6.1 Å, has slightly larger pore diameter than a usual cracking catalyst ZSM-5. The in-house synthetized ZSM-12 used in the experiments had the following textural properties: Si/AI molar ratio 40; BET area 313 m²/g; micropore area 241 m²/g; external area 71 m²/g; micropore volume 0.12 cm³/g; mesopore volume 0.14 cm³/g; crystal size 0.1 microns.

BEA type zeolite, such as BETA, is a zeolite with 3D channel system, 12-membered rings and pore openings of 7.5x6.3 Å and 6.9x6.0 Å. Beta zeolite having Si/AI molar ratio 34; BET area 546 m²/g; micropore area 353 m²/g; external area 191 m²/g; micropore volume 0.97 cm³/g; mesopore volume 0.17 cm³/g; crystal size 0.1-0.3 microns, was used in the experiments.

FAU type zeolite, such as USY, is a zeolite with 3D channel system, 12-membered rings and pore openings of 7.4x7.4 Å. USY zeolite having Si/AI molar ratio **24.24 UCS;** BET area 546 m²/g; micropore area 353 m²/g; external area 191 m²/g; micropore volume 0.97 cm³/g; mesopore volume 0.17 cm³/g; crystal size 0.3-0.5 microns, was used in the experiments.

IWV type zeolite, such as ITQ-27, is a zeolite with 2D channel system, 12-membered rings and pore openings of 6.2×6.9 Å. ITQ-27 zeolite having Si/AI molar ratio 17; BET area 468 m²/g; micropore area 418 m²/g; external area 50 m²/g; micropore volume 0.21 cm³/g; mesopore volume 0.08 cm³/g; crystal size 0.4-1.0 microns, was used in the experiments.

MSE type zeolite, such as MCM-68, is a zeolite with 3D channel system, 12- and 10-membered rings and pore openings of 6.4x6.8 Å, 5.2x5.8 Å and 5.2x5.2 Å. MCM-68 zeolite having Si/AI molar ratio 14; BET area 371 m²/g; micropore area 340 m²/g; external area 41 m²/g; micropore volume 0.14 cm³/g; mesopore volume 0.04 cm³/g; crystal size 0.1-0.3 microns, was used in the experiments.

IMF type zeolite, such as IM-5, is a zeolite with 2D channel system, 10-membered rings and pore openings of 5.3x5.9 Å and 4.8x5.4 Å. IM-5 zeolite having Si/AI molar ratio 14; BET area 302 m²/g; micropore area 275 m²/g; external area 27 m²/g; micropore volume 0.12 cm³/g; mesopore volume 0.06 cm³/g; crystal size 0.2-0.3 microns, was used in the experiments, synthesized following a hydrothermal method. Hierarchical IM-5 having Si/AI molar ratio 15; BET area 360 m²/g; micropore area 331 m²/g; external area 29 m²/g; micropore volume 0.24 cm³/g; mesopore volume 0.13 cm³/g; crystal size 0.2-0.4 microns, was used in the experiments, synthesized following the same hydrothermal method, but with addition of cellulose nanofibers to the hydrogel in a weight ratio of 0.4 Cellulose/Si resulting in a hierarchical structure with both micro- and mesopores.

### Catalytic cracking

Experiments were carried out using a continuous plug flow reactor system including a 19 mm diameter quartz tube where one or various catalytic beds can be stacked and heated by a 3 independent heating zones furnace controlled by internal thermocouples. In a typical experiment, previously calcined commercial or in-house synthetized catalysts were pelletized to obtain a desired particle size (i.e. 50-100 µm) to avoid any diffusion limitations and were diluted with silicon carbide to reach a constant volume of 5 cm³ for each one of the tested catalytic beds. At the top of the catalytic bed, an additional volume of 2 cm³ of pure silicon carbide was added, helping for the good vaporization of the injected feedstock before reaching the catalytic zone. Before each reaction, the system was preheated at the reaction temperature and was purged with 50 mL/min of nitrogen flow during at least 30 min. During the reaction step, feedstock was injected at a controlled flow to adjust the Weight Hourly Space Velocity (WHSV) of the reaction considering the amount of catalyst previously loaded. At the same time, a controlled nitrogen flow is introduced in the reactor to adjust the initial hydrocarbon partial pressure. Typically, nitrogen flow was adjusted to obtain a hydrocarbon partial pressure of 0.33 atm. Samplings at different Time-On-Stream (TOS) to determine the evolution of the activity and selectivity of the tested catalyst(s) were carried out. Liquid products were collected at the exit of the reactor in glass receivers kept at 289 K by means of a cold bath, while gaseous products were collected in a gas bag. Nitrogen was used as an internal standard for the quantification of the gaseous fraction. Gaseous products were analysed using a Shimadzu GC- 2014 chromatograph equipped with three detectors: two thermal conductivity detectors (TCD) for analysis of H₂ and N₂, after separation in a 6 ft MS 5A and 2.5 ft MS 13X molecular sieve respectively, and a flame ionization detector (FID) for C1-C6 hydrocarbons which were separated in a 164 ft plot/Al₂O₃ column. Liquid products were weighted and analysed through comprehensive two-dimensional gas chromatography (GC×GC). An Agilent 7890A GC equipped with a capillary flow technology GC×GC flow modulator (Agilent Technologies), a split injector at 250°C (250/1) (1.0 µL) and a flame ionization detection (FID) system (300 °C, 200 Hz). The column sets consisted of a combination of a non-polar column (HP-5 30 m × 0.25 mm × 0.5 µm, Agilent technologies) and a polar column (HP-INNOWax 5 m × 0.25 mm × 0.15 µm, Agilent technologies), placed in different ovens. The system was operated at a constant flow of 0.5 ml/min for the first column and 30 ml/min for the second one, and at programmed temperatures, going from 50 °C (1 min) to 260 °C (79 min) at 3 °C/min. High purity hydrogen was used as a carrier gas at constant flow rate. Modulation period was set to 4.5 s and data were processed using the GC image^{™} v2.1 software developed by GC Image^{™}, LLC. At the end of the reaction, stripping was carried out during 15 minutes with a high nitrogen flow to remove all the hydrocarbons of the system before to carry out the regeneration step where 100 mL/min of air was injected at a temperature of 550 °C. Coke was estimated by measuring the CO₂ produced during the catalyst regeneration step, by means of an infrared analyser. This procedure was used in the following Examples.

### Example 1 - Influence of Si/AI molar ratio and cracking temperature over catalyst activity and light olefins selectivity during cracking of pure alkanes, as illustrated with ZSM-5 catalysts having different Si/AI molar ratios

Experiments were conducted at 400 °C, 500 °C and 600 °C, atmospheric pressure, and constant initial partial pressure of hydrocarbons of 0.33 atm through nitrogen dilution, space velocity was varied to obtain different conversion levels and to compare product selectivity with special emphasis in propylene. Influence of WHSV conditions over the cracking activity in terms of conversion and propylene yield for ZSM-5 catalysts having different Si/AI molar ratio and similar other textural properties for the processing of n-hexadecane feed at different temperatures is shown in Figures 3 and 4. Figure 3 illustrates influence of the Si/AI molar ratio of the ZSM-5 catalyst over catalyst activity at 400 ºC (A), 500 ºC (B) and 600 ºC (C), when cracking pure n-hexadecane at total pressure of 1 atm, feed's partial pressure of 0.33 atm, and varying WHSV. Figure 4 illustrates influence of the cracking temperature over catalyst activity and propylene selectivity for the ZSM-5 catalysts having different Si/AI molar ratio of 31 (A and B), 125 (C and D) and 400 (E and F) when cracking pure n-hexadecane at total pressure of 1 atm, feed's partial pressure of 0.33 atm, and varying WHSV.

From Figure 3 it can be seen that the lower the Si/AI molar ratio of the used ZSM-5 catalyst, the more active the catalysts were for cracking pure n-hexadecane.

On the other hand, cracking temperature has also an impact. When cracking n-hexadecane in the range of 400-600 ºC, it can be seen from Figure 4; A, C and E that the higher the temperature the higher the conversion at a given space velocity, independently of the used ZSM-5 catalyst. Propylene selectivity is also positively affected when the cracking temperature is increased (Fig. 4; B, D and F).

Based on the results of these tests, also shown in Table 2, it can be concluded that an elevated cracking temperature, a solid acid catalyst having limited Si/AI molar ratio, and an elevated space velocity (limited mean residence time), appear to be beneficial contributors for reaching high conversion levels and for maximizing the yields of light olefins and propylene. These findings on the temperature, mean residence time and Si/AI molar ratio are applicable also to the first and the second cracking stage of the present method.

**Table 2. Product yields at maximum propylene yield when cracking pure n-hexadecane in a fixed-bed reactor at Pₜₒₜ = 1 atm, P_{0Feed} = 0.33 atm.**

| | ZSM-5 (31) | | | ZSM-5 (125) | | | ZSM-5 (400) | | |
|---|---|---|---|---|---|---|---|---|---|
| Trx (ºC) | 400 | 500 | 600 | 400 | 500 | 600 | 400 | 500 | 600 |
| WHSV (h⁻¹) | 391 | 197 | 198 | 153 | 198 | 98 | 2.5 | 2.5 | 2.5 |
| Conversion (wt.%) | 92.1 | 98.8 | 97.4 | 93.8 | 96.1 | 98.3 | 97.7 | 98.6 | 96.5 |
| C1-C4 paraffins (wt.%) | 16.2 | 21.1 | 11.9 | 18.8 | 18.0 | 13.8 | 21.7 | 17.6 | 16.3 |
| \| Methane (wt.%) | 0.0 | 0.2 | 1.2 | 0.0 | 0.2 | 1.5 | 0.0 | 0.5 | 5.5 |
| \| Ethane (wt.%) | 0.0 | 0.5 | 1.9 | 0.0 | 0.4 | 2.1 | 0.1 | 1.1 | 5.0 |
| \| Propane (wt.%) | 6.7 | 10.3 | 4.9 | 7.8 | 8.9 | 6.0 | 9.4 | 8.6 | 3.6 |
| \| Butanes (wt.%) | 9.5 | 10.1 | 3.9 | 11.0 | 8.5 | 4.2 | 12.2 | 7.4 | 2.2 |
| C2-C4 olefins (wt.%) | 29.4 | 50.5 | 63.6 | 28.6 | 50.2 | 64.6 | 28.0 | 48.3 | 61.6 |
| \| Ethylene (wt.%) | 0.5 | 5.3 | 11.8 | 0.8 | 3.6 | 13.4 | 1.2 | 5.6 | 12.8 |
| \| Propylene (wt.%) | 11.0 | 24.1 | 32.9 | 10.7 | 23.7 | 33.3 | 10.8 | 23.1 | 31.8 |
| \| Butenes (wt.%) | 17.9 | 21.1 | 18.9 | 17.1 | 22.9 | 17.9 | 16.0 | 19.6 | 17.0 |
| C2=/C3= | 0.05 | 0.22 | 0.45 | 0.07 | 0.15 | 0.40 | 0.11 | 0.24 | 0.42 |
| HTI (C4s=/C4s) | 1.9 | 2.1 | 5.0 | 1.6 | 2.7 | 4.4 | 1.3 | 2.7 | 7.6 |
| C5+ (wt.%) | 54.2 | 28.2 | 24.1 | 52.5 | 31.6 | 21.1 | 49.7 | 33.4 | 19.1 |
| \| Paraffins (wt.%) | 22.9 | 11.6 | 14.3 | 21.2 | 15.5 | 11.2 | 17.5 | 16.4 | 8.8 |
| \| Olefins (wt.%) | 20.6 | 9.5 | 4.9 | 18.5 | 11.2 | 4.3 | 14.8 | 8.8 | 3.7 |
| \| Cycloalkanes (wt.%) | 9.2 | 3.6 | 1.4 | 10.2 | 2.9 | 1.5 | 12.3 | 4.4 | 1.1 |
| \| Cyclic olefins (wt.%) | 0.7 | 1.0 | 0.6 | 0.9 | 0.6 | 0.5 | 1.2 | 0.8 | 0.6 |
| \| Aromatics-BTX (wt.%) | 0.7-0.3 | 2.3-1.5 | 2.6-2.1 | 1.6-0.7 | 1.1-0.7 | 3.4-2.8 | 3.7-1.4 | 2.8-1.8 | 4.5-3.4 |
| \| Unknowns (wt.%) | 0.1 | 0.2 | 0.3 | 0.1 | 0.3 | 0.2 | 0.2 | 0.2 | 0.4 |
| Coke (wt.%) | 0.2 | 0.2 | 0.4 | 0.1 | 0.2 | 0.5 | 0.6 | 0.7 | 3.0 |

### Example 2 - Usability of ZSM-12 and ZSM-5 especially in the first catalytic cracking

Experiments were conducted using ZSM-5 (31) or ZSM-12 (40) zeolites, at 600 °C, atmospheric pressure and 0.33 atm of initial partial pressure of hydrocarbons (diluted in N₂), space velocity was varied in order to obtain different conversion levels. Influence of WHSV conditions over cracking activity in terms of conversion and propylene yield for both catalysts using n-paraffinic and isomerized feeds is shown in Figure 5.

After checking that, in the case of the n-paraffinic feed R1, the catalytic activity is not controlled by diffusion limitations, it can be clearly observed in (A) of Figure 5 that ZSM-5 catalyst is a more active material than ZSM-12 to crack high molecular weight linear paraffins as higher conversion levels can be reached under the same severity conditions. Despite that both materials have similar Si/AI molar ratio, and comparable acidic properties, the higher cracking activity of ZSM-5 catalyst is very likely due to a more constrained environment enhancing the confinement effect and consequently the cracking rate of hydrocarbons. On the other hand, it is also worth observing that, in the case of linear paraffins, higher propylene yields and light olefins in general may be obtained using ZSM-5 as seen in (B) of Figure 5. This is believed to be due to the shape selectivity of the ZSM-5 zeolite which limit the occurrence of bimolecular reactions and, in particular, secondary hydrogen transfer reactions, which limit the production of light olefins, as naphthenes formed by dimerization-cyclization of olefins donate hydrogen to other olefins and become aromatics while the olefins are hydrogenated into paraffins. Paraffins are more stable than olefins, partially explaining the lower rate of re-cracking of the ZSM-12 catalyst, even if part of the lower cracking activity of the ZSM-12 catalyst could be also explained by diffusion effects. In addition, re-cracking of saturated hydrocarbons compared to olefins is less selective to light olefins explaining also partially the slightly lower selectivity to propylene of ZSM-12 zeolite compared to ZSM-5.

However, in the case of the iso-paraffinic feedstock R2, ZSM-12 having one-dimensional channel system of 12-ring channels (5.7 × 6.1 Å) is more active compared to ZSM-5 zeolite, which has three-dimensional channel system of 10-ring channels (5.2 × 5.7 and 5.3 × 5.6 Å channels), highlighting the importance of mass transport issues and accessibility when branched hydrocarbons are processed ((A) in Figure 5). Although the maximum propylene yield provided by ZSM-5 was slightly higher with the n-paraffinic feed in a once-through setup compared to ZSM-12, in turn, for isomerized feed ZSM-12 provides significantly higher maximum propylene yield than ZSM-5 ((B) in Figure 5). The low catalytic activity of ZSM-5 when branched hydrocarbons are processed implies to apply much lower WHSV or higher mean residence times to reach high conversion levels and propylene yields, leading to an important increase of the kinetic rate of secondary mechanisms and in particular of secondary hydrogen transfer reactions explaining, in a large extent, the much lower light olefins and propylene selectivity obtained when ZSM-5 is used to crack the isomerized feed. On the contrary, in the case of the ZSM-12, as lower mass transport issues occur, very similar WHSV conditions may be applied to obtain high conversion levels and propylene yields, independently of the processed feedstock, explaining why ZSM-12 is a more flexible catalyst in terms of feedstock processability to efficiently and selectively produce light olefins and propylene from linear and branched hydrocarbons cracking in a fixed-bed reactor.

Altogether, it can be concluded that ZSM-12 zeolite is a more active and selective catalyst to convert the highly isoparaffinic feed in the first catalytic cracking to propylene and light olefins. As it can be observed in Table 3, ZSM-12 catalyst brings the advantage to reach high conversion levels and maximum propylene yield independently of the processed feedstock, unlike ZSM-5. ZSM-5's conversion of n-paraffinic feed is about 1.5-3 times the conversion of isoparaffinic feed at the same WHSV as already observed in (A) of Figure 5. Both zeolites generated more propylene than ethylene, but the propylene to ethylene weight-ratios were clearly higher with ZSM-12 compared to ZSM-5. ZSM-5 zeolite promotes the olefinicity of C2 fraction, i.e. ratio of ethylene to total C2, for both feedstocks at maximum propylene yield, around 0.9 versus 0.8 for ZSM-5 and ZSM-12 catalyst, respectively as seen in Table 3, while ZSM-12 catalyst promotes the olefinicity of the C3 fraction, i.e. ratio of propylene to total C3, around 0.9 versus 0.8 for ZSM-12 and ZSM-5 catalyst, respectively. The yields of propylene, C4 mono-olefins and ethylene provided by ZSM-12 were of similar level for both feeds, and propylene and C4 mono-olefins yields were much higher than those provided by ZSM-5 at maximum propylene yield. Similar low coke yields at the maximum propylene yield are obtained for both catalysts and feedstocks.

**Table 3. Product yields at maximum propylene yield when cracking n-paraffinic or iso-paraffinic feedstock in a fixed-bed reactor at Trx = 600 °C, Pₜₒₜ = 1 atm, P_{0Feed} = 0.33 atm and using ZSM-5 (31) or ZSM-12 (40) zeolites in the first catalytic cracking.**

| | ZSM-5 (31) | | ZSM-12 (41) | |
|---|---|---|---|---|
| Processed feedstock | n-paraffinic | iso-paraffinic | n-paraffinic | iso-paraffinic |
| Cracking temperature (ºC) | 600 | 600 | 600 | 600 |
| Conversion (wt.%) | 97.8 | 72.0 | 93.4 | 84.4 |
| C1-C4 paraffins (wt.%) | 13.5 | 13.8 | 9.1 | 8.8 |
| \| Methane (wt.%) | 0.9 | 1.1 | 2.1 | 1.5 |
| \| Ethane (wt.%) | 1.4 | 1.2 | 2.5 | 1.2 |
| \| Propane (wt.%) | 6.3 | 6.2 | 2.1 | 2.7 |
| \| Butanes (wt.%) | 4.9 | 5.3 | 2.4 | 3.4 |
| C2-C4 olefins (wt.%) | 65.9 | 40.7 | 63.4 | 54.9 |
| \| Ethylene (wt.%) | 12.1 | 10.9 | 8.1 | 5.9 |
| \| Propylene (wt.%) | 34.0 | 20.2 | 28.3 | 28.0 |
| \| Butenes (wt.%) | 19.8 | 9.6 | 27.0 | 21.0 |
| C2=/C3= | 0.36 | 0.54 | 0.29 | 0.21 |
| C2 olefinicity | 0.90 | 0.91 | 0.76 | 0.83 |
| C3 olefinicity | 0.84 | 0.77 | 0.93 | 0.91 |
| HTI (C4s=/C4s) | 4.0 | 1.8 | 12.2 | 6.4 |
| C5+ (wt.%) | 20.2 | 45.1 | 27.0 | 35.8 |
| \| n-Paraffins (wt.%) | 10.1 | 19.6 | 11.6 | 6.0 |
| \| iso-Paraffins (wt.%) | 1.2 | 15.2 | 0.9 | 18.8 |
| \| Olefins (wt.%) | 5.0 | 3.1 | 12.8 | 6.9 |
| \| Cycloalkanes (wt.%) | 1.0 | 1.0 | 0.6 | 1.1 |
| \| Cyclic olefins (wt.%) | 0.8 | 0.6 | 0.3 | 0.5 |
| \| Aromatics-BTX (wt%) | 1.8-1.4 | 5.4-4.5 | 0.6-0.2 | 2.3-1.3 |
| \| Unknowns (wt.%) | 0.3 | 0.2 | 0.2 | 0.2 |
| C5+ Olefinicity | 0.27 | 0.08 | 0.49 | 0.21 |
| Coke (wt.%) | 0.4 | 0.4 | 0.5 | 0.5 |

Typically, the relative activity of cracking catalysts for generating secondary reactions is estimated using a hydrogen transfer index (HTI). Catalysts with higher HTI (expressed in the present disclosure as a weight ratio of unsaturated C4 to saturated C4) are thought to generate fewer secondary reactions, preserving a greater quantity of C5+ olefins, that can be subsequently recycled and cracked to lighter olefins. In order to maximise propylene, it is important to suppress secondary hydrogen transfer by maximizing the availability of olefin precursors. Secondary hydrogen transfer reactions may involve the formation of bulky bimolecular reaction intermediates, and are believed to be controlled by steric constraints, due to the space available inside the micropores of the zeolites. They can also occur on the outer surface of the zeolite particles. Generally, it has been thought that the smaller the pore size of the zeolite, the greater the extent of the suppression of the hydrogen transfer reactions of the alkenes, meaning that the HTI should increase with decreased pore size of the zeolite. However, ZSM-12 is able to convert both the n-paraffinic and the highly iso-paraffinic feeds to propylene with excellent conversion levels, and with less secondary reactions than ZSM-5 as shown in Table 3, where ZSM-12 provides much higher HTI values than ZSM-5 at maximum propylene yield. It is believed that even though the smaller channel size of ZSM-5 may limit access of bigger feed molecules, the crossing points in its 3D interconnecting channel system may compensate this to some extent, and provide more space for reactions, compared to zeolites having similar channel size but a channel system of lower dimensionality. Additionally, the composition of the C5+ fraction has significant impact on its crackability in the second catalytic cracking, for example high olefinicity of the C5+ fraction enhances greatly its crackability: C5+ olefins are easier to crack compared to paraffins having same carbon number, and upon cracking each C5+ olefin molecule produces 2 molecules of lighter olefins, while a paraffin molecule produces only 1 molecule of light olefin and light paraffin. Both zeolites provided good olefinicity to the C5+ fraction, especially ZSM-12. Also, a low total content of cyclics, i.e. aromatics and naphthenes, particularly a low total content of aromatics, is desired for cracking in the second stage, as naphthenes convert relatively easily to aromatics, and aromatics may increase coking and are relatively inert and hence not likely to convert to the desired products. Both ZSM-5 and ZSM-12 generated sufficiently controlled levels C1-C4 paraffins and aromatics which are difficult to convert to desired products in the second cracking. When ZSM-12 is used in the first catalytic cracking, around 55-60 wt.% of light olefins are obtained already in the first catalytic cracking, and about one-third of the feed can still be converted in the second catalytic cracking. When ZSM-5 is used in the first catalytic cracking, around 65 wt.% of light olefins are obtained already in the first catalytic cracking, and about 20 wt.% of the feed can still be converted in the second catalytic cracking, when using highly n-paraffinic feed. However, when using highly isoparaffinic feed, a much bigger fraction of C5+ hydrocarbons, about 40 wt.%, can still be converted in the second catalytic cracking. Hence, there are certain opportunities to utilise even much smaller reactors in the second catalytic cracking.

### Example 3 - Two-stage catalytic cracking of n-paraffinic feed using ZSM-5 in the first cracking, and same ZSM-5 (comparative) or ZSM-12 in the second cracking of C5+ fraction of the first catalytically cracked stream in a separate reactor

Intensification of the propylene production is illustrated here by subjecting to a second catalytic cracking at least part of the first catalytically cracked stream which has not been cracked enough during the first pass (i.e. C5+ fraction) to give additional C2-C4 products and especially light olefins. It is worth mentioning that such intensification may not be achieved merely by increasing the mean residence time in a single reactor as it has been clearly demonstrated that the increase of the mean residence time during the catalytic cracking of a sustainable hydrocarbon feed greatly promotes the occurrence of secondary reaction such as secondary hydrogen transfer reactions leading to significantly decreased yields of light olefins at the benefit of light paraffins and aromatic compounds. It is thus of interest to subject at least part of a first catalytically cracked stream to a second catalytic cracking. In the experiments light olefins stream was separated from the C5+ fraction at the exit of a first fixed-bed cracking reactor working under operative conditions which maximize the production of propylene and light olefins, and the C5+ fraction was then fed to a second fixed-bed reactor allowing the selective re-cracking into the desired olefinic products. Compared to direct recycling, the two-stage catalytic cracking process brings more flexibility in terms of selection of catalyst and operative conditions including the possibility to tune the cracking temperature and space velocity of the re-cracking of the C5+ fraction allowing to deeper maximize the production of light olefins coming from the C5+ fraction re-cracking. Generally, the concept tested here is as shown in Figure 2.

In the case of the n-paraffinic feed, high conversion levels and light olefins yields may be achieved in the first catalytic cracking stage by using ZSM-5 (10 MR) or ZSM-12 (12 MR) as the first zeolite, and by using ZSM-12 (12 MR) or ZSM-5 (10 MR) as the second zeolite, respectively, beneficial conditions may be achieved for further converting the molecules of the first catalytically cracked stream to the desired cracking products, with reduced coking and/or side product formation. For comparison purposes, a two-stage cracking using the same ZSM-5 zeolite in both stages was also conducted.

A single-pass run was thus conducted with ZSM-12 or ZSM-5 catalysts processing the n-paraffinic feed as in the previous examples at 600 °C, atmospheric pressure and 0.33 bar of initial hydrocarbon partial pressure. WHSV for each one of the used zeolites has been optimized to maximize the production of propylene during this first cracking step corresponding to 250 and 25 h-1 for respectively ZSM-5 and ZSM-12 zeolite. Enough C5+ fractions have been recovered and accumulated to optimize the second cracking in the second reactor and to maximize the production of the light olefins and propylene for each one of the considered configurations. Table 4 shows the obtained results for the configuration involving the use of ZSM-5 as the first cracking catalyst, while results concerning configuration using ZSM-12 as the first cracking catalyst are reported in Example 4, Table 5.

**Table 4. Product yields at maximum propylene yield when subjecting n-paraffinic feedstock to a first cracking in a fixed-bed reactor using ZSM-5 (31) followed by second cracking of C5+ fraction at maximum propylene yield in a second fixed bed cracking reactor using ZSM-5 (31; comparative) or ZSM-12 (40) zeolites.**

| | 1^{st} cat cracking | 2^{nd} cat cracking of C5+ | | WHOLE PROCESS | |
|---|---|---|---|---|---|
| Catalyst | ZSM-5 (31) | ZSM-5 (31) comparative | ZSM-12 (40) | ZSM-5 (1^{st}) - ZSM-5 (2^{nd}) comparative | ZSM-5 (1^{st}) - ZSM-12 (2^{nd}) |
| Feedstock | n-paraffinic feed | C5+ fraction | C5+ fraction | n-paraffinic feed | n-paraffinic feed |
| WHSV (h⁻¹) | 250 | 25 | 25 | 250 (1^{st}) - 25 (2^{nd}) | 250 (1^{st}) - 25 (2^{nd}) |
| Cracking temperature (ºC) | 600 | 600 | 600 | 600 (1^{st}) - 600 (2^{nd}) | 600 (1^{st}) - 600 (2^{nd}) |
| Conversion (wt.%) | 97.8¹ | 76.1² | 63.2² | 99.5¹ | 99.8¹ |
| C1-C4 paraffins (wt.%) | 13.5 | 27.4 | 9.9 | 19.0 | 15.4 |
| \| Methane (wt.%) | 0.8 | 2.0 | 1.1 | 1.2 | 1.1 |
| \| Ethane (wt.%) | 1.4 | 3.0 | 1.4 | 2.1 | 1.7 |
| \| Propane (wt.%) | 6.4 | 14.0 | 3.7 | 9.1 | 7.0 |
| \| Butanes (wt.%) | 4.9 | 8.4 | 3.7 | 6.6 | 5.6 |
| C2-C4 olefins (wt.%) | 65.9 | 48.7 | 53.3 | 75.6 | 76.6 |
| \| Ethylene (wt.%) | 12.1 | 16.2 | 9.5 | 15.4 | 14.1 |
| \| Propylene (wt.%) | 34.0 | 22.9 | 27.0 | 38.6 | 39.5 |
| \| Butenes (wt.%) | 19.8 | 9.6 | 16.8 | 21.6 | 23.0 |
| C2=/C3= | 0.36 | 0.71 | 0.35 | 0.39 | 0.35 |
| C2 olefinity | 0.90 | 0.84 | 0.87 | 0.88 | 0.89 |
| C3 olefinity | 0.84 | 0.62 | 0.88 | 0.81 | 0.85 |
| HTI (C4s=/C4s) | 4.0 | 1.1 | 5.5 | 3.3 | 4.2 |
| C5+ (wt.%) | 20.1 | 22.9 | 36.0 | 4.8 | 7.4 |
| \| n-Paraffins (wt.%) | 10.1 | 3.2 | 14.8 | 0.6 | 3.0 |
| \| iso-Paraffins (wt.%) | 1.2 | 2.5 | 6.1 | 0.5 | 1.2 |
| \| Olefins (wt.%) | 5.0 | 3.1 | 7.3 | 0.6 | 1.5 |
| \| Cycloalkanes (wt.%) | 1.0 | 0.9 | 1.5 | 0.2 | 0.3 |
| \| Cyclic olefins (wt.%) | 0.7 | 0.5 | 0.6 | 0.1 | 0.1 |
| \| Aromatics-BTX (wt.-%) | 1.8-1.4 | 12.6-11.1 | 5.4-4.1 | 2.6-2.2 | 1.1-0.8 |
| \| Unknowns (wt.%) | 0.3 | 0.1 | 0.3 | 0.2 | 0.2 |
| C5+ Olefinity | 0.25 | 0.13 | 0.20 | 0.12 | 0.20 |
| Coke (wt.%) | 0.5 | 1.0 | 0.8 | 0.6 | 0.6 |

| | | | | | |
|---|---|---|---|---|---|
| ¹ Defined as C1-C12 + coke yields. ² Defined as C1-C4 + coke yields. | | | | | |

From Table 4 it can be seen that the strategy to use the two-stage catalytic cracking process where the cracking of the initial n-paraffinic feedstock is optimized to maximize the production of propylene in a first reactor and the C5+ fraction is subsequently re-cracked with a different catalyst in a second fixed reactor, where the conditions are also optimized to maximize propylene, allows to increase the overall production of light olefins, including propylene, and to greatly reduce production of C1-C4 paraffins. Compared to using the same ZSM-5 catalyst in both stages, formation of propylene vs ethylene is enhanced, as can be seen from the C2=/C3= ratio, and the olefinicity of both C2 and C3 fraction can be increased. Significant improvements are seen in the C5+ fraction quality: its aromaticity is reduced and olefinicity significantly increased, thereby having higher value for recycling, compared to the concept using the same ZSM-5 catalyst in both stages.

### Example 4 - Two-stage catalytic cracking of n-paraffinic and isoparaffinic feeds using ZSM-12 in the first cracking, and ZSM-5 in recracking of C5+ fraction in a separate reactor

The first experiment of Example 4 is essentially repetition of experiments in Example 3, but just using ZSM-12 in the first catalytic cracking and ZSM-5 in the second catalytic cracking.

**Table 5. Product yields at maximum propylene yield when subjecting n-paraffinic feedstock to a first cracking in a fixed-bed reactor using ZSM-12 (40) followed by second cracking of C5+ fraction at maximum propylene yield in a second fixed bed cracking reactor using ZSM-5 (31).**

| | 1^{st} cat cracking | 2^{nd} cat cracking of C5+ | WHOLE PROCESS |
|---|---|---|---|
| Catalyst | ZSM-12 (40) | ZSM-5 (31) | ZSM-12 (1^{st}) - ZSM-5 (2^{nd}) |
| Feedstock | n-paraffinic feed | C5+ fraction | n-paraffinic feed |
| WHSV (h⁻¹) | 25 | 75 | 25 (1^{st}) - 25 (2^{nd}) |

| Cracking temperature (ºC) | 600 | 600 | 600 (1^{st}) - 600 (2^{nd}) |
|---|---|---|---|
| Conversion (wt.%) | 93.4¹ | 70.8² | 99.3¹ |
| C1-C4 paraffins (wt.%) | 8.9 | 8.2 | 11.3 |
| \| Methane (wt.%) | 2.0 | 1.0 | 2.2 |
| \| Ethane (wt.%) | 2.5 | 1.5 | 2.9 |
| \| Propane (wt.%) | 2.1 | 3.2 | 3.2 |
| \| Butanes (wt.%) | 2.3 | 2.5 | 3.0 |
| C2-C4 olefins (wt.%) | 63.4 | 62.5 | 80.4 |
| \| Ethylene (wt.%) | 8.1 | 15.1 | 12.2 |
| \| Propylene (wt.%) | 28.3 | 31.9 | 37.0 |
| \| Butenes (wt.%) | 27.0 | 15.5 | 31.2 |
| C2=/C3= | 0.29 | 0.48 | 0.33 |
| C2 olefinity | 0.76 | 0.91 | 0.84 |
| C3 olefinity | 0.93 | 0.91 | 0.92 |
| HTI (C4s=/C4s) | 12.2 | 6.0 | 10.4 |
| C5+ (wt.%) | 26.3 | 29.1 | 7.7 |
| \| n-Paraffins (wt.%) | 11.6 | 14.8 | 3.9 |
| \| iso-Paraffins (wt.%) | 0.9 | 4.4 | 1.1 |
| \| Olefins (wt.%) | 12.8 | 3.6 | 0.9 |
| \| Cycloalkanes (wt.%) | 0.6 | 0.9 | 0.2 |
| \| Cyclic olefins (wt.%) | 0.3 | 0.6 | 0.2 |
| \| Aromatics-BTX (wt.%) | 0.9-0.2 | 4.7-3.0 | 1.3-0.8 |
| \| Unknowns (wt.%) | 0.1 | 0.1 | 0.1 |
| C5+ Olefinity | 0.49 | 0.12 | 0.12 |
| Coke (wt.%) | 0.5 | 0.2 | 0.6 |

| | | | |
|---|---|---|---|
| ¹ Defined as C1-C12 + coke yields. ² Defined as C1-C4 + coke yields. | | | |

From the results it can be seen that the strategy to use the two-stage catalytic cracking process where the cracking of the initial n-paraffinic feedstock is optimized to maximize the production of propylene in a first reactor, and the C5+ fraction is subsequently re-cracked with a different catalyst in a second fixed reactor, where the conditions are also optimized to maximize propylene, allows to greatly increase the overall production of light olefins. In this way it is possible to increase the light olefins yield by 10-15 wt.%, reaching light olefins yields as high as 80 wt.%, and the propylene yield by 5-10 wt.%, leading to propylene yields as high as 40 wt.%. It is worth mentioning that the strategy to use ZSM-12 as first cracking catalyst, brings the further advantage to produce less light paraffins and aromatics and to be more selective to light olefins, keeping at the same time a higher yield of recyclable C5+ fractions from the second reactor, which could be recycled to the first or second reactor to further increase the light olefin yields.

Concerning the iso-paraffinic feed R2 having very high isoparaffin content (93 wt.%) and a high content of very bulky multibranched isoparaffins (50 wt.%), ZSM-5 as a 10 MR catalyst is not the best suitable catalyst to maximize conversion and light olefins yield due to diffusion limitations. Therefore only the ZSM-12 catalyst was tested in the first cracking followed by a second cracking of the C5+ fraction with a ZSM-5 catalyst. In the same way as in Example 2, this strategy was experimentally tested and a single-pass run was conducted with ZSM-12 catalyst processing the iso-paraffinic feed at 600 °C, atmospheric pressure and 0.33 bar of initial hydrocarbon partial pressure. WHSV was optimized to maximize the production of propylene during this first cracking step corresponding to 40 h-1. Enough of the C5+ fraction was recovered and accumulated to optimize the second catalytic cracking and to maximize the production of the light olefins and propylene. Table 6 shows the obtained results. As it can be appreciated, in the case of the very highly isoparaffinic feed, the two-stage catalytic cracking process strategy allows also to greatly improve the light olefins and propylene yields by around 15 and 8 wt.%, respectively, without increasing extensively the light paraffins and aromatic compounds throughput. It may be observed that the conversion of the iso-paraffinic feed into C1-C4 hydrocarbons is more refractory than the n-paraffinic feed, leading to higher yield of remaining C5+ fraction even after a second cracking (i.e. around 17 wt.%). Recycling of the remaining C5+ fraction to the first or second catalytic cracking is thus an interesting option when processing the iso-paraffinic feedstock to further increase the light olefin yields.

**Table 6. Product yields at maximum propylene yield when subjecting an iso-paraffinic feedstock to a first cracking in a fixed-bed reactor using ZSM-12 (40) followed by second cracking of C5+ fraction at maximum propylene yield in a second fixed bed cracking reactor using ZSM-5 (31).**

| | 1^{st} cat cracking | 2^{nd} cat cracking of C5+ | WHOLE PROCESS |
|---|---|---|---|
| Catalyst | ZSM-12 (40) | ZSM-5 (31) | ZSM-12 (1^{st}) - ZSM-5 (2^{nd}) |
| Feedstock | Iso-paraffinic feed | C5+ fraction | Iso-paraffinic feed |
| WHSV (h⁻¹) | 40 | 25 | 40 (1^{st}) - 25 (2^{nd}) |
| Cracking temperature (ºC) | 600 | 600 | 600 |
| Conversion (wt.%) | 84.4¹ | 53.4² | 93.4¹ |
| C1-C4 paraffins (wt.%) | 8.8 | 8.7 | 11.8 |
| \| Methane (wt.%) | 1.5 | 1.9 | 2.0 |
| \| Ethane (wt.%) | 1.2 | 2.1 | 2.0 |
| \| Propane (wt.%) | 2.7 | 3.0 | 3.8 |
| \| Butanes (wt.%) | 3.4 | 1.7 | 4.0 |
| C2-C4 olefins (wt.%) | 54.8 | 44.0 | 70.6 |
| \| Ethylene (wt.%) | 5.8 | 12.5 | 10.3 |
| \| Propylene (wt.%) | 28.0 | 21.9 | 35.8 |
| \| Butenes (wt.%) | 21.0 | 9.6 | 24.5 |
| C2=/C3= | 0.21 | 0.57 | 0.29 |
| C2 olefinity | 0.83 | 0.85 | 0.84 |
| C3 olefinity | 0.91 | 0.88 | 0.91 |
| HTI (C4s=/C4s) | 6.4 | 5.7 | 6.1 |
| C5+ (wt.%) | 35.9 | 46.6 | 16.8 |
| \| n-Paraffins (wt.%) | 6.1 | 10.2 | 3.7 |
| \| iso-Paraffins (wt.%) | 18.8 | 23.7 | 8.5 |
| \| Olefins (wt.%) | 6.9 | 3.9 | 1.4 |
| \| Cycloalkanes (wt.%) | 1.1 | 1.0 | 0.4 |
| \| Cyclic olefins (wt.%) | 0.5 | 0.7 | 0.2 |
| \| Aromatics - BTX (wt.%) | 2.3-1.3 | 6.9-5.8 | 2.4-2.1 |
| \| Unknowns (wt.%) | 0.2 | 0.2 | 0.2 |
| C5+ Olefinity | 0.19 | 0.08 | 0.08 |
| Coke (wt.%) | 0.5 | 0.7 | 0.8 |

| | | | |
|---|---|---|---|
| ¹ Defined as C1-C12 + coke yields. ² Defined as C1-C4 + coke yields. | | | |

### Example 5 - Usability of beta-zeolite and USY especially in the first catalytic cracking

In this example, use of beta-zeolite or USY in the first catalytic cracking is illustrated with the highly isoparaffinic feed. Both beta and USY have the largest ring-size 12 MR, and 3D channel system. Comparison was made to ZSM-5 (10 MR, 3D). Experiments were conducted at 600 °C, atmospheric pressure, and constant initial partial pressure of hydrocarbons of 0.33 atm through nitrogen dilution, space velocity was varied between 10 to 5000 h-1 in order to obtain different conversion levels. Influence of WHSV conditions over the cracking activity in terms of conversion for beta and USY, compared to ZSM-5, for the processing of the highly isoparaffinic feed is shown in Figure 6A and 6B. Also the relative compositions of C5+ fractions obtained using beta (C5+ yield: 49 wt.%) and USY (C5+ yield: 45 wt.%) were analysed, reflecting crackability of the C5+ fraction in the second catalytic cracking. Both the USY zeolite and the beta-zeolite provided high olefinicity to the C5+ fraction (27 wt.% and 40 wt.%, respectively) as well as paraffins content (14wt.% n-paraffins & 47 wt.% isoparaffins, and 15 wt.% n-paraffins & 38 wt.% isoparaffins wt.%, respectively), with somewhat elevated but still controlled content of aromatics (8 wt.% and 4 wt.%, respectively). Hence both of these zeolites are good candidates for use in the present method, particularly in the first catalytic cracking, and especially for cracking feeds containing bulky molecules.

### Example 6 - Usability of hierarchical IM-5 especially in the first catalytic cracking

In this example, use of hierarchical IM-5 in the first catalytic cracking is illustrated with the n-paraffinic and highly isoparaffinic feeds. IM-5 has a framework type having the largest ring-size 10 MR and 2D channel system, and producing it as hierarchical does not change these. However, as can be seen from the catalyst list in the foregoing, the mesoporisation increased BET area, micropore area and volume, and mesopore volume.

First, a comparison was made between the hierarchical IM-5, the parent IM-5 and an otherwise similar IM-5 but with higher Si/AI molar ratio (26) when cracking the n-paraffinic feed. Experiments were conducted at 600 °C, atmospheric pressure, and constant initial partial pressure of hydrocarbons of 0.33 atm through nitrogen dilution, space velocity was varied between 25 to 1500 h-1 in order to obtain different conversion levels. The results are reported in Figure 7, showing that utilising the hierarchical IM-5 increased the yield of propylene by about 4-5 wt.% and the yield of light olefins (C2-C4) a bit more, compared to the parent IM-5 at a similar conversion level. This highlights that the increased mesoporosity in the hierarchical IM-5 compared to the parent IM-5 and the IM-5 with the higher Si/AI molar ratio allows to decrease the hydrogen transfer mechanism resulting in an increased yield of the light olefins.

Next, a comparison was made between the hierarchical IM-5 and ZSM-5 (31), USY, beta, ITQ-27, MCM-68 and ZSM-12 when cracking the highly isoparaffinic feed. Experiments were conducted at 600 °C, atmospheric pressure, and constant initial partial pressure of hydrocarbons of 0.33 atm through nitrogen dilution, space velocity was varied between 0 to 2000 h-1 in order to obtain different conversion levels. The results are reported in Figure 8, which shows that the feed-flexibility of the 10 MR zeolite was enhanced when mesoporised, exhibiting a higher conversion compared to the conventionally used ZSM-5 zeolite, when cracking the isoparaffinic feed. Besides the high conversion rate, the hierarchical IM-5 also showed a high selectivity (full results not reported here) towards propene and light (C2-C4) olefins (26.9 wt.% and 52.8 wt.%, respectively). This was second best after ZSM-12 (28.0 wt.% and 54.9 wt.%, respectively), and clearly exceeding the other tested zeolites. The rather high fraction of C5+ (33.5 wt.%) provided by the hierarchical IM-5 as well as ZSM-12 confirms that significant propylene yields could be achieved in the second cracking stage. The composition of the C5+ fraction provided by the hierarchical IM-5 is similar to the ZSM-12 for isoparaffins (about 50 wt.% of the C5+ fraction), and paraffins (-22-25 wt.%), while the content of olefins provided by the hierarchical IM-5 compared to ZSM-12 is much lower (-10 wt.% vs ~20 wt.%) and aromatics higher (-10 wt.% vs 4.3 wt.%). This evidences the usability of the C5+ fraction in the second cracking stage, preferably after aromatics removal.

Additionally, a further comparison was made between the hierarchical IM-5, ZSM-5 (31), and ZSM-12 when cracking the n-paraffinic feed. Experiments were conducted at 600 °C, atmospheric pressure, and constant initial partial pressure of hydrocarbons of 0.33 atm through nitrogen dilution, at space velocity 250 h-1 (ZSM-5), 150 h-1 (hierarchical IM-5) and 25 h-1 (ZSM-12). The results of the relative compositions at equilibrium of the C5+ fractions are reported in Table 7, showing the beneficial higher olefinicity of the C5+ fractions, as well as higher aromatics contents, obtained with the hierarchical IM-5 and ZSM-12, compared to ZSM-5. Upon closer analysis of the aromatics rich fractions, results of which are also reported in Table 7, it can be seen, that the share of BTX obtained with the hierarchical IM-5 and ZSM-12 is over 90 wt.%, and that the hierarchical IM-5 exhibits rather high selectivity towards xylene.

**Table 7. Comparison of the relative compositions of C5+ fractions obtained with ZSM-5, ZSM-12 and hierarchical IM-5, when cracking n-paraffinic feed at 600 °C, atmospheric pressure, and feed's initial partial pressure of 0.33 atm, and WHSV 250 h-1 (ZSM-5), 150 h-1 (hierarchical IM-5) or 25 h-1 (ZSM-12), as well as comparison of the relative composition of the aromatics fractions separated from the C5+ fractions.**

| | ZSM-5 | ZSM-12 | Hierachical IM-5 |
|---|---|---|---|
| C5+ fraction relative composition at equilibrium state | | | |
| Cyclic compounds excl. aromatics (wt.-%) | 5.3 | 5.0 | 5.1 |
| Olefins (wt.-%) | 13.7 | 25.5 | 18.3 |
| Isoparaffins (wt.-%) | 9.3 | 14.7 | 18.3 |
| n-paraffins (wt.-%) | 65.6 | 40.8 | 34.9 |
| Aromatics (wt.-%) | 6.1 | 14.0 | 23.4 |

| Relative composition of the aromatics in the C5+ fractions at equilibrium state | | | |
|---|---|---|---|
| Benzene (wt.-%) | 12.4 | 6.9 | 5.8 |
| Toluene (wt.-%) | 39.5 | 29.2 | 29.3 |
| Xylenes (wt.-%) | 27.1 | 27.1 | 39.7 |
| Other alkyl-aromatics (wt.-%) | 21.0 | 36.9 | 25.2 |

Finally, catalyst resistance to deactivation was assessed based on a coke-on-catalyst (CoC) parameter, when using the highly isoparaffinic feed. ITQ-27 exhibited the lowest deactivation constant, hierarchical IM-5 being the second best before beta and ZSM-12.

As a conclusion, with the hierarchical IM-5 both n-paraffinic and highly isoparaffinic feeds can be cracked to propene and other light olefins without sacrificing the product yield, suggesting hierarchical 10 MR zeolites as good candidates especially for the first zeolite. Additionally, the hierarchical IM-5 provided increased aromatics content in the C5+ fraction exhibiting a rather high selectivity towards xylene. Hence, an aromatics rich fraction could be separated from the C5+ fraction, not only for reducing the coke-forming load on the subsequent catalyst, but for increasing the pool of valuable products recoverable from the present method.

### Comparative Example 7 - Two-stage catalytic cracking of n-paraffinic feed using ZSM-5(31) in the first catalyst bed and ZSM-5(125) or ZSM-5(31) in the second catalyst bed

Catalytic cracking tests with n-hexadecane representing an n-paraffinic feed were conducted using ZSM-5(31) at 400 °C and ZSM-5(125) at 600 °C and at different space velocities. The catalytic cracking reaction conditions and products of each experiment are shown in Table 8. The WHSV was varied to obtain different conversion levels. This was done by adjusting the feed rate and/or the catalyst weight.

**Table 8. Results (mass-balance normalized) from a two-stage catalytic cracking of n-hexadecane using ZSM-5(31) in the first cracking followed by a second cracking with ZSM-5(125), without separation in-between. As comparison results from two-stage catalytic cracking of n-paraffinic feed using ZSM-5(31) in the first cracking, followed by cracking C5+ fraction with ZSM-5(31), are provided.**

| Processed feedstock | n-hexadecane | n-paraffinic feed |
|---|---|---|
| 1^{st} and 2^{nd} catalyst | ZSM-5(31)-ZSM-5(125) | ZSM-5(31)-ZSM-5(31) (C5+ fraction to 2^{nd} cracking, only) |
| Cracking temperature (ºC) | 1st 400ºC; 2nd 600ºC | 1st 600ºC; 2nd 600ºC |

| WHSV | 1st ∼400; 2nd ∼600 | 1st ∼600; 2nd ∼600 |
|---|---|---|
| Conversion (wt.%) | 95.2 | 76.1² |
| C1-C4 paraffins (wt.%) | 16.7 | 27.4 |
| \| Methane (wt.%) | 1.5 | 2.0 |
| \| Ethane (wt.%) | 1.4 | 3.0 |
| \| Propane (wt.%) | 7.0 | 14.0 |
| \| Butanes (wt.%) | 6.8 | 8.4 |
| C2-C4 olefins (wt.%) | 53.2 | 48.7 |
| \| Ethylene (wt.%) | 4.6 | 16.2 |
| \| Propylene (wt.%) | 23.4 | 22.9 |
| \| n-Butenes (wt.%) | 13.9 | - |
| \| i-Butene (wt.%) | 10.6 | - |
| \| Butadiene (wt.%) | 0.7 | - |
| \| Total Butenes (wt.%) | 25.2 | 9.6 |
| C3=/C2= | 5.1 | 1.4 |
| C2 olefinicity | 0.77 | 0.84 |
| C3 olefinicity | 0.77 | 0.62 |
| HTI (C4s=/C4s) | 3.7 | 1.1 |
| C5+ (wt.%) | 29.1 | 22.9 |
| \| n-Paraffins (wt.%) | 12.3 | 3.2 |
| \| iso-Paraffins (wt.%) | - | 2.5 |
| \| Olefins (wt.%) | 14.2 | 3.1 |
| \| Cycloalkanes (wt.%) | 1.4 | 0.9 |
| \| Cyclic olefins (wt.%) | 0.7 | 0.5 |
| \| Total Aromatics (wt%) | 0.8 | 12.6 |
| \| BTX (wt%) | 0.4 | 11.1 |
| \| Unknowns (wt.%) | 0.5 | 0.1 |
| C5+ Olefinicity | 0.51 | 0.13 |
| Coke (wt.%) | 0.1 | 1.0 |

From these results it can be seen that using in the second cracking a zeolite having the same framework type but different, in this case higher, Si/AI molar ratio, compared to the first zeolite, i.e. first ZSM-5(31) and secondly ZSM-5(125), yield of light olefins - also propylene yield - may be increased, and C1-C4 paraffin yield reduced, compared to using same zeolite, here ZSM-5(31), in both cracking stages.This comparative example shows that when both the first and the second zeolite are of 10 MR framework type, mere adjustment of the Si/AI molar ratio of the zeolites does not provide sufficient improvement. When using a (non-hierarchical) zeolite of 10 MR framework type as the first zeolite, the second zeolite should be selected so that it has more space within its structure, so that the mass transport limitations of the first zeolite could be compensated, and feed-flexibility of the method ensured.

Various embodiments have been presented. It should be appreciated that in this document, words comprise, include and contain are each used as open-ended expressions with no intended exclusivity.

The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed embodiments of this invention may be used to advantage without the corresponding use of other features. As such, the foregoing description shall be considered as merely illustrative of the principles of the present invention, and not in limitation thereof. Hence, the scope of the invention is only restricted by the appended patent claims.

## Claims

1. A method for producing propylene, the method comprising the following steps:
a) providing a sustainable hydrocarbon feed;
b) subjecting a first cracking feed comprising the sustainable hydrocarbon feed to catalytic cracking reaction in the presence of a first cracking catalyst comprising a first zeolite at a temperature within a range from 300 to 700 °C, preferably from 400 to 650 °C, to obtain a first catalytically cracked stream;
c) optionally separating from the first catalytically cracked stream at least a lighter fraction comprising at least C1-C3 hydrocarbons and a heavier fraction;
d) subjecting a second cracking feed comprising at least a portion, preferably the heavier fraction, of the first catalytically cracked stream to catalytic cracking reaction in the presence of a second cracking catalyst comprising a second zeolite at a temperature within a range from 350 to 750 °C, preferably from 450 to 700 °C, to obtain a second catalytically cracked stream; and
e) recovering from the second catalytically cracked stream, and optionally from the lighter fraction of the first catalytically cracked stream, at least a fraction rich in propylene;
wherein:
the first zeolite is selected from a group consisting of zeolites of a framework type having 10-membered ring as the largest ring-size (10 MR framework type), hierarchical zeolites of a 10 MR framework type, and zeolites of a framework type having 12-membered ring as the largest ring-size (12 MR framework type), preferably from hierarchical zeolites of a 10 MR framework type and zeolites of a 12 MR framework type,
the second zeolite is selected from a group consisting of zeolites of a 10 MR framework type, hierarchical zeolites of a 10 MR framework type, and zeolites of a 12 MR framework type, and
the second zeolite has a framework type that is different from the first zeolite unless both the first and the second zeolite are hierarchical zeolites of a 10 MR framework type,
with the proviso that when the first zeolite is a zeolite of a 10 MR framework type, the second zeolite is a hierarchical zeolite of a 10 MR framework type or a zeolite of a 12 MR framework type.

2. The method according to claim 1, wherein the heavier fraction separated from the first catalytically cracked stream comprises hydrocarbons having a carbon number of at least C4, preferably at least C5, and optionally the heavier fraction is subjected to an aromatics removal and/or to a selective hydrogenation of diolefins to mono-olefins before incorporating into the second cracking feed.

3. The method according to claim 1 or 2, wherein the sustainable hydrocarbon feed comprises, based on the total weight of the sustainable hydrocarbon feed, at least 50 wt.%, preferably at least 60 wt.%, more preferably at least 70 wt.% paraffins, even more preferably at least 90 wt.-% paraffins, preferably having a carbon number of at least C12; and/or at least 20 wt.%, preferably at least 40 wt.%, more preferably at least 60 wt.%, even more preferably at least 80 wt.% isoparaffins; and/or at least 10 wt.%, preferably at least 20 wt.%, more preferably at least 30 wt.%, even more preferably at least 40 wt.% multibranched isoparaffins; and/or at most 40 wt.%, preferably at most 30 wt.%, more preferably at most 20 wt.%, even more preferably at most 10 wt.% mono-olefins.

4. The method according to any one of claims 1 to 3, wherein the sustainable hydrocarbon feed has a T5 temperature (EN ISO 3405-2019) at least 180 °C, preferably at least 190 °C, more preferably at least 200 °C, and a T95 temperature (EN ISO 3405-2019) at most 600 °C, preferably at most 550°C, more preferably at most 500 °C; and/or T5 and T95 temperatures (EN ISO 3405-2019), preferably an initial boiling point and a final boiling point, within 180°C - 65 0°C, preferably within 190 °C - 600 °C, more preferably within 200 °C - 550°C.

5. The method according to any one of claims 1 to 4, wherein the sustainable hydrocarbon feed has a difference between the T95 and T5 temperatures (EN ISO 3405-2019), preferably a difference between an initial boiling point and a final boiling point, at most 300 °C, preferably at most 200 °C, more preferably at most 150 °C, even more preferably at most 100 °C, further more preferably at most 80 °C.

6. The method according to any one of claims 1 to 5, wherein the sustainable hydrocarbon feed comprises, based on the total weight of the sustainable hydrocarbon feed, at most 15 wt.%, preferably at most 10 wt.%, more preferably at most 5 wt.%, even more preferably at most 1 wt.% aromatics; and/or at most 30 wt.%, preferably at most 25 wt.%, more preferably at most 10 wt.%, even more preferably at most 5 wt.% naphthenes; and/or at most 3 wt.%, preferably at most 2 wt.%, more preferably at most 1 wt.%, even more preferably at most 0.5 wt.% oxygenated hydrocarbons, expressed as elemental oxygen (ASTMD5622-2017).

7. The method according to any one of claims 1 to 6, wherein the first zeolite and the second zeolite have a Si/AI molar ratio, selected independently from each other, within a range from 5 to 250, preferably from 5 to 150, more preferably from 10 to 100.

8. The method according to any one of claims 1 to 7, wherein
the second zeolite is selected from zeolites of a 10 MR framework type, hierarchical zeolites of a 10 MR framework type, and zeolites of a 12 MR framework type, having largest pore diameter smaller than the largest pore diameter in the framework type of the first zeolite, preferably from zeolites of a 10 MR framework type and hierarchical zeolites of a 10 MR framework type,
the second zeolite is selected so that the largest pore diameter in the framework type is smaller than the largest pore diameter in the framework type of the first zeolite,
the first zeolite is of a framework type wherein the channel system dimensionality is higher compared to the channel system dimensionality of the second zeolite,
the first and the second zeolite are hierarchical zeolites of a 10 MR framework type, preferably hierarchical zeolites of the same 10 MR framework type, more preferably hierarchical zeolites of the same 10 MR framework type and mesoporosity, and/or
the first and the second zeolite are of the same framework type, but the second zeolite has a lower Si/AI molar ratio compared to the first zeolite.

9. The method according to any one of claims 1 to 8 wherein the first zeolite is of a framework type MFI, IMF, ITH, FER, MWW, TON, MTT, MTW, BEA, FAU, MSE or IWV, preferably of a framework type MFI, IMF, MTW, BEA or FAU, and preferably the second zeolite is of a framework type MFI, IMF, ITH, FER, MWW, TON, MTT, MTW, BEA, FAU, MSE, or IWV, preferably of a framework type MFI, IMF, MTW, BEA, or FAU.

10. The method according to any one of claims 1 to 9 comprising vaporizing the first and/or the second cracking feed before subjecting to the catalytic cracking reaction.

11. The method according to any one of claims 1 to 10 wherein the total pressure at step b) and step d) is selected, independently from each other, within a range from 50 kPa to 150 kPa (absolute), preferably from 80 kPa to 120 kPa (absolute), more preferably step b) and step d) are conducted at about atmospheric pressure.

12. The method according to any one of claims 1 to 11 wherein the mean residence time of the second cracking feed in step d) is shorter than the mean residence time of the first cracking feed in step b).

13. The method according to any one of claims 1 to 12 wherein step e) further comprises recovering from the second catalytically cracked stream, and optionally from the lighter fraction of the first catalytically cracked stream, a fraction rich in ethylene, a fraction rich in one or more of C4 mono-olefins, and/or a fraction rich in saturated C1-C4 hydrocarbons; and/or recovering from the second catalytically cracked stream a fraction of hydrocarbons having a carbon number of at least C4, preferably at least C5, and/or a fraction rich in aromatics.

14. The method according to any one of claims 1 to 13, wherein the first and/or the second cracking feed comprises, based on the total weight of the cracking feed, from 0.5 wt.% to 50 wt.%, preferably from 0.5 wt.% to 30 wt.%, more preferably from 1 wt.% to 20 wt.% a diluent gas, optionally comprising at least one or more of steam, methane, ethane, propane, nitrogen, carbon monoxide, carbon dioxide, and/or saturated C1-C4 hydrocarbons separated from the first and/or the second catalytically cracked stream.

15. The method according to any one of claims 1 to 14, wherein the first cracking feed comprises, based on the total weight of the first cracking feed, at least 40 wt.%, preferably at least 50 wt.%, more preferably at least 60 wt.%, even more preferably at least 90 wt.% the sustainable hydrocarbon feed, preferably the remaining of the first cracking feed consisting of a diluent gas and/or a recycle stream of hydrocarbons having a carbon number of at least C4, preferably at least C5, separated from the first and/or the second catalytically cracked stream.

16. The method according to any one of claims 1 to 15 comprising separating from the second catalytically cracked stream a fraction of hydrocarbons having a carbon number of at least C4, preferably at least C5, and recycling at least a portion thereof to the first and/or the second cracking feed, preferably to the second cracking feed, optionally after subjecting to an aromatics removal and/or to a selective hydrogenation of diolefins to mono-olefins.

17. The method according to any one of claims 1 to 16, wherein the sustainable hydrocarbon feed comprises at least one or more of a hydrotreated and optionally hydroisomerised biomass feedstock, a hydrotreated and optionally hydroisomerised waste plastic liquefaction oil, and/or a diene-depleted selectively hydrogenated waste plastic liquefaction oil.

18. The method according to any one of claims 1 to 17, wherein in step b) the first cracking catalyst and/or in step d) the second cracking catalyst is arranged at least in one or more catalyst bed(s) in one or more reactor(s), preferably at least in one or more fluidised catalyst bed reactor(s) and/or fixed catalyst bed reactor(s).
